# EUROPEAN PATENT APPLICATION

(11) **EP 4 324 821 A1**
(43) Date of publication of application: **21.02.2024**
(21) Application number: 22795619.0
(22) Date of filing: 18.04.2022
(51) Int. Cl.: C07C 211/55, C07C 211/56, C08K 5/18, C08L 27/12, C07D 213/74

(54) **BISDIAMINOPHENYL COMPOUND, CROSSLINKING AGENT, COMPOSITION, AND MOLDED ARTICLE**

(30) Priority: 27.04.2021 JP 2021074899
(71) Applicant: DAIKIN INDUSTRIES, LTD., Osaka-Shi, Osaka 530-0001 (JP)
(72) Inventor: KANBARA, Tadashi, Osaka-Shi, Osaka 530-0001 (JP); NOGUCHI, Tsuyoshi, Osaka-Shi, Osaka 530-0001 (JP); KAMIYA, Fumihiro, Osaka-Shi, Osaka 530-0001 (JP); OTA, Daisuke, Osaka-Shi, Osaka 530-0001 (JP); SUDO, Atsushi, Higashiosaka-shi, Osaka 577-8502 (JP)
(74) Representative: Hoffmann Eitle
(86) International application number: PCT/JP2022/018031
(87) International publication number: WO 2022/230706

(57) **Abstract**

The disclosure aims to provide a novel compound that can be used as a cross-linking agent that provides a cross-linked product with excellent plasma resistance while maintaining heat resistance, or the like. Provided is a compound represented by the following formula wherein R¹ is -SO₂-, -O-, -CO-, an optionally substituted alkylene group, a group represented by the following formula or a single bond; and two As are each independently an aromatic ring group containing a 5- or 6-membered aromatic ring and optionally containing a substituent, where the aromatic ring group contains a substituent in the case where the aromatic ring group is a monocyclic aromatic ring group containing a 6-membered aromatic hydrocarbon ring.

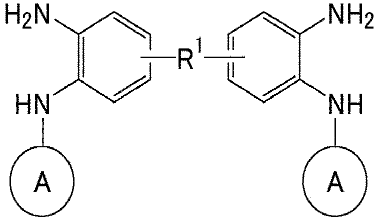

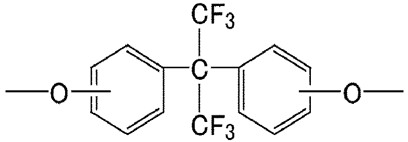

## Description

### TECHNICAL FIELD

The disclosure relates to bisdiaminophenyl compounds, cross-linking agents, compositions, and molded articles.

### BACKGROUND ART

Patent Literature 1 proposes a compound (A) containing at least two cross-linkable reactive groups (I) represented by the following formula (I): 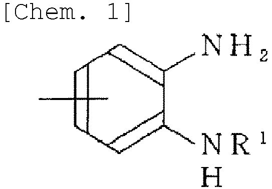 (wherein R¹s are the same as or different from each other, and are each a fluorine atom or a monovalent organic group) as a cross-linking agent for fluorine-containing elastomers.

### CITATION LIST

### - Patent Literature

Patent Literature 1: WO 01/032773 A

### SUMMARY OF INVENTION

### - Technical Problem

The disclosure aims to provide a novel compound that can be used as a cross-linking agent for providing a cross-linked product with excellent plasma resistance while maintaining heat resistance, and a cross-linking agent, a composition, and a molded article each containing the compound.

### - Solution to Problem

The disclosure relates to a compound represented by the following formula (1): 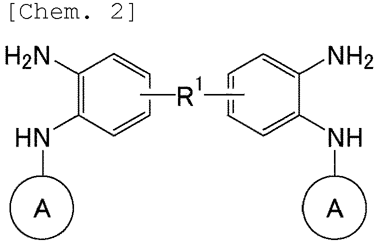 wherein
R¹ is -SO₂-, -O-, -CO-, an optionally substituted alkylene group, a group represented by the following formula: 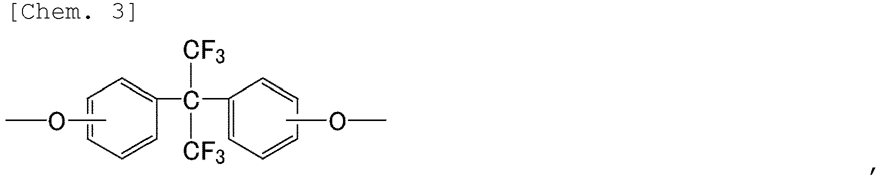 or a single bond; and
two As are each independently an aromatic ring group containing a 5- or 6-membered aromatic ring and optionally containing a substituent, where the aromatic ring group contains a substituent in the case where the aromatic ring group is a monocyclic aromatic ring group containing a 6-membered aromatic hydrocarbon ring.

Preferably, the aromatic ring group is a monocyclic aromatic ring group containing a 6-membered aromatic hydrocarbon ring and containing a substituent or a monocyclic aromatic ring group containing a 5- or 6-membered aromatic heterocycle containing a nitrogen atom or a sulfur atom.

Preferably, the aromatic ring group is a group having a greater n-electron deficiency than a benzene ring.

Preferably, the substituent includes at least one selected from the group consisting of a halogen atom and an alkyl group optionally containing a halogen atom.

The disclosure also relates to a cross-linking agent containing the compound.

The disclosure also relates to a composition containing a fluorine-containing elastomer and the compound or the cross-linking agent.

Preferably, the fluorine-containing elastomer contains a cross-linkable group, and the cross-linkable group includes at least one selected from the group consisting of a hydroxy group (-OH), a cyano group (-CN), a carboxyl group (-COOH), an alkoxycarbonyl group, and an acid halide group.

The disclosure also relates to a molded article obtainable by cross-linking the composition.

### - Advantageous Effects of Invention

The compound of the disclosure can be used as a cross-linking agent for providing a cross-linked product with excellent plasma resistance while maintaining heat resistance.

The cross-linking agent and composition of the disclosure can provide a cross-linked product with excellent plasma resistance while maintaining heat resistance.

The molded article of the disclosure has excellent plasma resistance while maintaining heat resistance.

### DESCRIPTION OF EMBODIMENTS

The disclosure will be specifically described below.

The disclosure relates to a compound represented by the following formula (1):

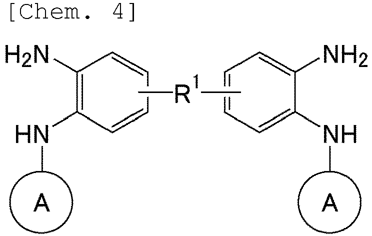

In the formula (1), R¹ is -SO₂-, -O-, -CO-, an optionally substituted alkylene group, a group represented by the following formula: 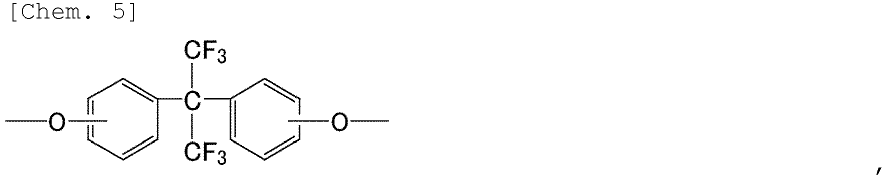 or a single bond.

Non-limiting preferred specific examples of the optionally substituted alkylene group include a C1-C6 non-substituted alkylene group and a C1-C10 perfluoroalkylene group. The perfluoroalkylene group is preferably a group represented by the following formula:

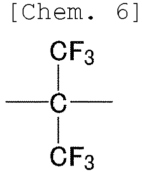

R¹ may be bonded to any position of both the right and left benzene rings. From the viewpoint of easy synthesis and easy progress of a cross-linking reaction, R¹ is preferably bonded such that either an NH₂ group or an amino group substituted with the aromatic ring group A is positioned at the para position.

In the formula (1), two As are each independently an aromatic ring group containing a 5- or 6-membered aromatic ring and optionally containing a substituent, where the aromatic ring group contains a substituent in the case where the aromatic ring group is a monocyclic aromatic ring group containing a 6-membered aromatic hydrocarbon ring.

When the compound in which As are each the above aromatic ring group is used as a cross-linking agent, the resulting cross-linked product can have better plasma resistance (in particular, oxygen plasma resistance) while maintaining heat resistance. The compound also can provide a cross-linked product with good compression set properties and good crack resistance. Moreover, the compound in which As are each the above aromatic ring group can have a higher melting point, which can industrially advantageously reduce contamination of the mold during molding.

The aromatic ring is preferably an aromatic hydrocarbon ring or an aromatic heterocycle.

When the aromatic ring group contains an aromatic hydrocarbon ring, it is preferably monocyclic or polycyclic, more preferably monocyclic.

The aromatic ring group containing an aromatic hydrocarbon ring does not include an aromatic ring group containing an aromatic heterocycle described later.

Specific examples of the aromatic ring group containing an aromatic hydrocarbon ring include a phenyl group, a naphthyl (e.g., 1-naphthyl, 2-naphthyl) group, a biphenyl (e.g., 2-biphenyl, 3-biphenyl, 4-biphenyl) group, and an anthryl (e.g., 2-anthryl) group.

The aromatic ring group containing an aromatic hydrocarbon ring may have one or more substituents. It should be noted that a monocyclic aromatic ring group (phenyl group) containing a 6-membered aromatic hydrocarbon ring needs to have a substituent.

The number of substituents is preferably 1 to 5, more preferably 1 to 3, still more preferably 1 or 2.

Examples of the substituent include a halogen atom (e.g., fluorine atom, chlorine atom) and an alkyl group optionally containing a halogen atom. The halogen atom is preferably a fluorine atom. The alkyl group preferably contains 1 to 5 carbon atoms, more preferably 1 or 2 carbon atoms, still more preferably 1 carbon atom.

The substituent preferably includes, among others, at least one selected from the group consisting of a halogen atom and an alkyl group optionally containing a halogen atom, more preferably at least one selected from the group consisting of a fluorine atom and an alkyl group optionally containing a fluorine atom, still more preferably at least one selected from the group consisting of a fluorine atom and a fluoroalkyl group, even more preferably at least one selected from the group consisting of a fluorine atom and a C1-C5 fluoroalkyl group, particularly preferably at least one selected from the group consisting of a fluorine atom and a trifluoromethyl group. The compound containing the above substituent can provide a cross-linked product with better properties in terms of heat resistance, plasma resistance, compression set properties, and crack resistance. Moreover, the compound containing the above substituent can have a higher melting point, which can industrially advantageously reduce contamination of the mold during molding.

In order to provide a cross-linked product with even better properties in terms of heat resistance, plasma resistance, compression set properties, and crack resistance, the aromatic ring group containing an aromatic hydrocarbon ring is preferably a monocyclic aromatic group containing a 6-membered aromatic hydrocarbon ring and containing a substituent. It is more preferably a monocyclic aromatic group containing a 6-membered aromatic hydrocarbon ring and containing at least one selected from the group consisting of a halogen atom and an alkyl group optionally containing a halogen atom. It is still more preferably a monocyclic aromatic ring group containing a 6-membered aromatic hydrocarbon ring and containing at least one selected from the group consisting of a fluorine atom and an alkyl group optionally containing a fluorine atom. It is even more preferably -C₆H₅₋ₙRfₙ (wherein Rf is a fluorine atom or a fluoroalkyl group; and n is an integer of 1 to 5) or -C₆H₅₋ₘRₘ (wherein R is an alkyl group; and m is an integer of 1 to 5). It is particularly preferably - C₆H₅₋ₙRfₙ (wherein Rf is a fluorine atom or a fluoroalkyl group; and n is an integer of 1 to 5).

The aromatic ring group containing an aromatic hydrocarbon ring is also preferably -C₆H₅₋ₘRₘ (wherein R is an alkyl group; and m is an integer of 1 to 5).

In these embodiments, the substituent may be positioned at any of the ortho-, meta-, and para-positions relative to the nitrogen atom to which A binds in the formula (1). For ease of synthesis, it may be positioned at the ortho- or meta-position relative to the nitrogen atom to which A binds in the formula (1).

The number of carbon atoms in the fluoroalkyl group for Rf is preferably 1 to 5, more preferably 1 or 2, still more preferably 1.

The fluoroalkyl group for Rf is preferably a trifluoromethyl group.

Rf is preferably positioned at the ortho- or meta-position relative to the nitrogen atom to which A binds in the formula (1).

n is preferably an integer of 1 to 3, more preferably 1 or 2, still more preferably 2.

The alkyl group for R preferably does not contain a substituent such as a fluorine atom. The number of carbon atoms in the alkyl group is preferably 1 to 5, more preferably 1 or 2, still more preferably 1. R is preferably positioned at the ortho- or meta-position, more preferably at the ortho-position, relative to the nitrogen atom to which A binds in the formula (1).

m is preferably an integer of 1 to 3, more preferably 1 or 2, more preferably 1.

The aromatic heterocycle contains one or more heteroatoms. The heteroatom preferably includes at least one selected from the group consisting of a nitrogen atom, an oxygen atom, and a sulfur atom, preferably at least one selected from the group consisting of a nitrogen atom and a sulfur atom, still more preferably a nitrogen atom.

The aromatic ring group containing the aromatic heterocycle is preferably monocyclic or a condensed-ring group, more preferably monocyclic. When the aromatic ring group is a condensed-ring group, it may have a structure in which aromatic heterocycles are condensed with each other or a structure in which an aromatic heterocycle and an aromatic hydrocarbon ring are condensed. When the aromatic ring group is a condensed-ring group, it may be bicyclic, tricyclic, or the like, preferably bicyclic.

The aromatic ring group containing the aromatic heterocycle may or may not contain a substituent. Examples of the substituent include an alkyl group, a fluoroalkyl group, a fluorine atom, and a chlorine atom. The substituent preferably includes at least one selected from the group consisting of a fluorine atom and a fluoroalkyl group, more preferably at least one selected from the group consisting of a fluorine atom and a C1-C5 fluoroalkyl group, still more preferably at least one selected from the group consisting of a fluorine atom and a trifluoromethyl group.

The aromatic ring group containing an aromatic heterocycle is preferably an aromatic ring group containing a 5- or 6-membered aromatic heterocycle containing a nitrogen atom or a sulfur atom, more preferably a 6-membered aromatic heterocycle containing a nitrogen atom, still more preferably a monocyclic or bicyclic aromatic ring group containing a 6-membered aromatic heterocycle containing a nitrogen atom, particularly preferably a monocyclic aromatic group containing a 6-membered aromatic heterocycle containing a nitrogen atom.

The aromatic ring group containing an aromatic heterocycle is preferably a pyridyl, pyrazinyl, pyrimidinyl, triazinyl, quinolyl, or quinoxalinyl group, more preferably a pyridyl, pyrazinyl, pyrimidinyl, or triazinyl group, still more preferably a pyridyl group.

The aromatic ring group is preferably a group having a greater n-electron deficiency (having a lower n-electron density) than a benzene ring (phenyl group containing no substituent). When the compound of the disclosure containing the group is used as a cross-linking agent, the cross-linked structure exhibits n-electron deficiency, so that the resulting cross-linked product has excellent oxidation resistance. Moreover, the compound of the disclosure used as a cross-linking agent can provide a cross-linked product with even better properties in terms of heat resistance, plasma resistance, compression set properties, and crack resistance.

The group having a greater n-electron deficiency than a benzene ring is preferably -C₆H₅₋ₙRfₙ (wherein Rf is a fluorine atom or a fluoroalkyl group; and n is an integer of 1 to 5) or an aromatic ring group containing a 6-membered aromatic heterocycle containing a nitrogen atom.

It is more preferably -C₆H₅₋ₙRfₙ (wherein Rf is a fluorine atom or a C1-C5 fluoroalkyl group; and n is an integer of 1 to 5) or a monocyclic or bicyclic aromatic ring group containing a 6-membered aromatic heterocycle containing a nitrogen atom.

It is still more preferably -C₆H₅₋ₙRfₙ (wherein Rf is a fluorine atom or a C1-C5 fluoroalkyl group; and n is an integer of 1 to 5) or a monocyclic aromatic ring group containing a 6-membered aromatic heterocycle containing a nitrogen atom.

It is even more preferably -C₆H₅₋ₙRfₙ (wherein Rf is a fluorine atom or a trifluoromethyl group; and n is an integer of 1 to 5), a pyridyl group, a pyrazinyl group, a pyrimidinyl group, or a triazinyl group.

It is particularly preferably -C₆H₅₋ₙRfₙ (wherein Rf is a fluorine atom or a trifluoromethyl group; and n is an integer of 1 to 5) or a pyridyl group.

The aromatic ring group may be a group having a greater n-electron excess (having a higher n-electron density) than a benzene ring (phenyl group containing no substituent). When the compound of the disclosure containing the above group is used as a cross-linking agent, the cross-linking rate (T90) can be improved, which can promote cross-linking. Even when the compound is used together with a cross-linking agent containing the group having a greater n electron deficiency than a benzene ring, cross-linking can be promoted.

The group having a greater n-electron excess than a benzene ring is preferably -C₆H₅₋ₘRₘ wherein R is an alkyl group; and m is an integer of 1 to 5.

It is more preferably -C₆H₅₋ₘRₘ wherein R is a C1-C5 alkyl group; and m is an integer of 1 to 5.

It is still more preferably -C₆H₅₋ₘRₘ wherein R is a methyl group (-CH₃); and m is an integer of 1 to 5.

The alkyl group does not contain a substituent.

In order to provide a cross-linked product with even better properties in terms of heat resistance, plasma resistance, compression set properties, and crack resistance, the compound is preferably a compound represented by the following formula (1-1): 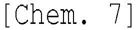 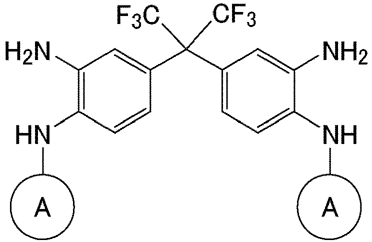 wherein A is as defined above.

It is more preferably a compound represented by the formula (1-1) wherein two As are each independently a monocyclic aromatic ring group containing a 6-membered aromatic hydrocarbon ring and containing a substituent, or a monocyclic aromatic ring group containing a 5- or 6-membered aromatic heterocycle containing a nitrogen atom or a sulfur atom.

It is still more preferably a compound represented by the formula (1-1) wherein two As are each independently - C₆H₅₋ₙRfₙ (wherein Rf is a fluorine atom or a fluoroalkyl group; and n is an integer of 1 to 5), -C₆H₅₋ₘRₘ (wherein R is an alkyl group; and m is an integer of 1 to 5), or an aromatic ring group containing a 6-membered aromatic heterocycle containing a nitrogen atom.

It is even more preferably a compound represented by the formula (1-1) wherein two As are each independently - C₆H₅₋ₙRfₙ (wherein Rf is a fluorine atom or a C1-C5 fluoroalkyl group; and n is an integer of 1 to 5) or a monocyclic or bicyclic aromatic ring group containing a 6-membered aromatic heterocycle containing a nitrogen atom.

It is further preferably a compound represented by the formula (1-1) wherein two As are each independently - C₆H₅₋ₙRfₙ (wherein Rf is a fluorine atom or a C1-C5 fluoroalkyl group; and n is an integer of 1 to 5) or a monocyclic aromatic ring group containing a 6-membered aromatic heterocycle containing a nitrogen atom.

It is particularly preferably a compound represented by the formula (1-1) wherein two As are each independently -C₆H₅₋ₙRfₙ (wherein Rf is a fluorine atom or a trifluoromethyl group; and n is an integer of 1 to 5), a pyridyl group, a pyrazinyl group, a pyrimidinyl group, or a triazinyl group.

In order to promote cross-linking, the compound is preferably a compound represented by the formula (1-1) wherein two As are each independently -C₆H₅₋ₘRₘ (wherein R is an alkyl group; and m is an integer of 1 to 5).

It is more preferably a compound represented by the formula (1-1) wherein two As are each independently -C₆H₅₋ₘRₘ (wherein R is a C1-C5 alkyl group; and m is an integer of 1 to 5).

It is still more preferably a compound represented by the formula (1-1) wherein two As are each independently - C₆H₅₋ₘRₘ (wherein R is a methyl group (-CH₃); and m is an integer of 1 to 5).

Preferred examples of the compound of the disclosure include compounds represented by the following formulas. In each formula, Rf represents a fluorine atom or a fluoroalkyl group, preferably a fluorine atom or a C1-C5 fluoroalkyl group, more preferably a fluorine atom or a trifluoromethyl group.

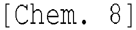

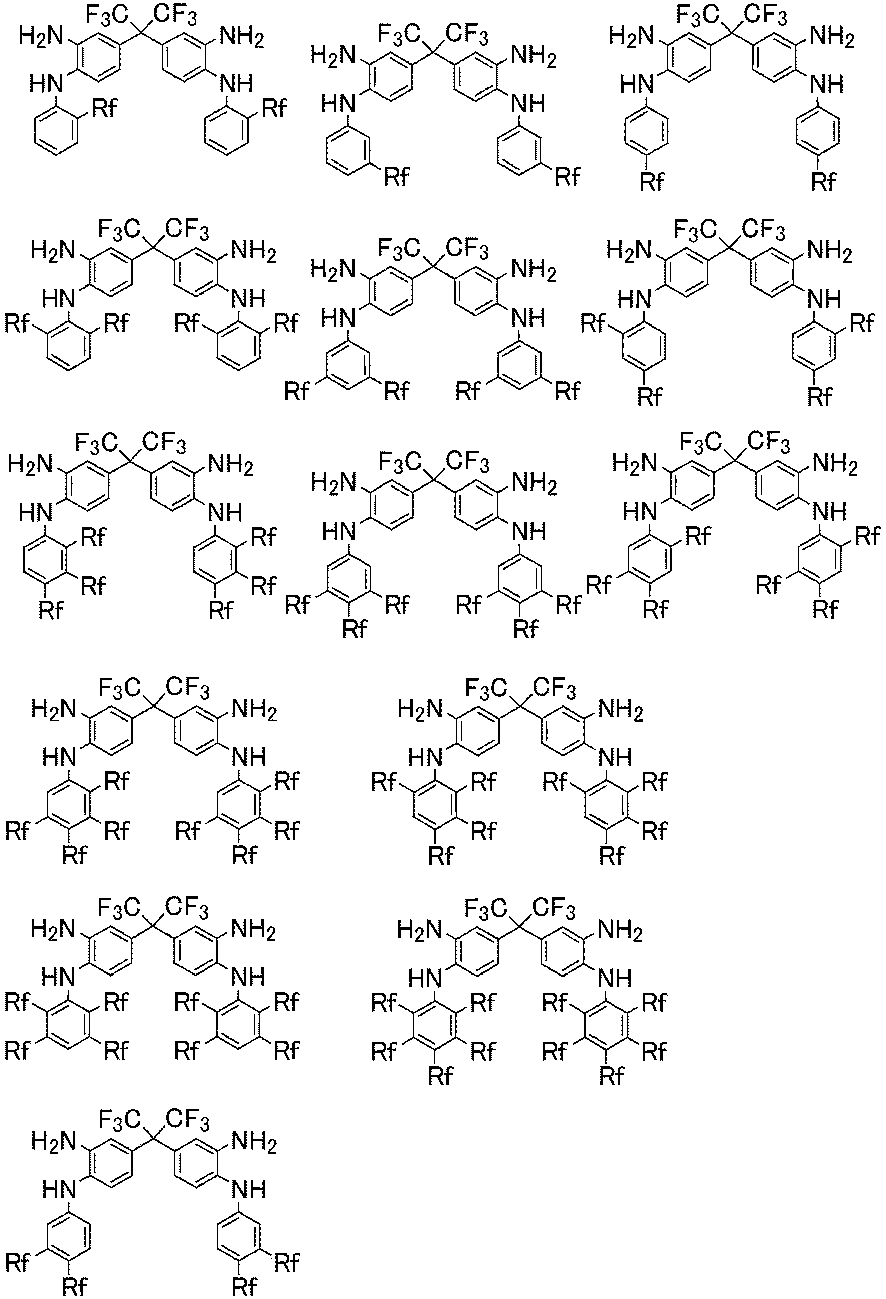

Preferred examples of the compound of the disclosure also include compounds represented by the following formulas. Each of the following compounds may contain a substituent represented by Rf (Rf represents a fluorine atom or a fluoroalkyl group, preferably a fluorine atom or a C1-C5 fluoroalkyl group, more preferably a fluorine atom or a trifluoromethyl group) on the heterocycle.

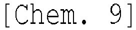

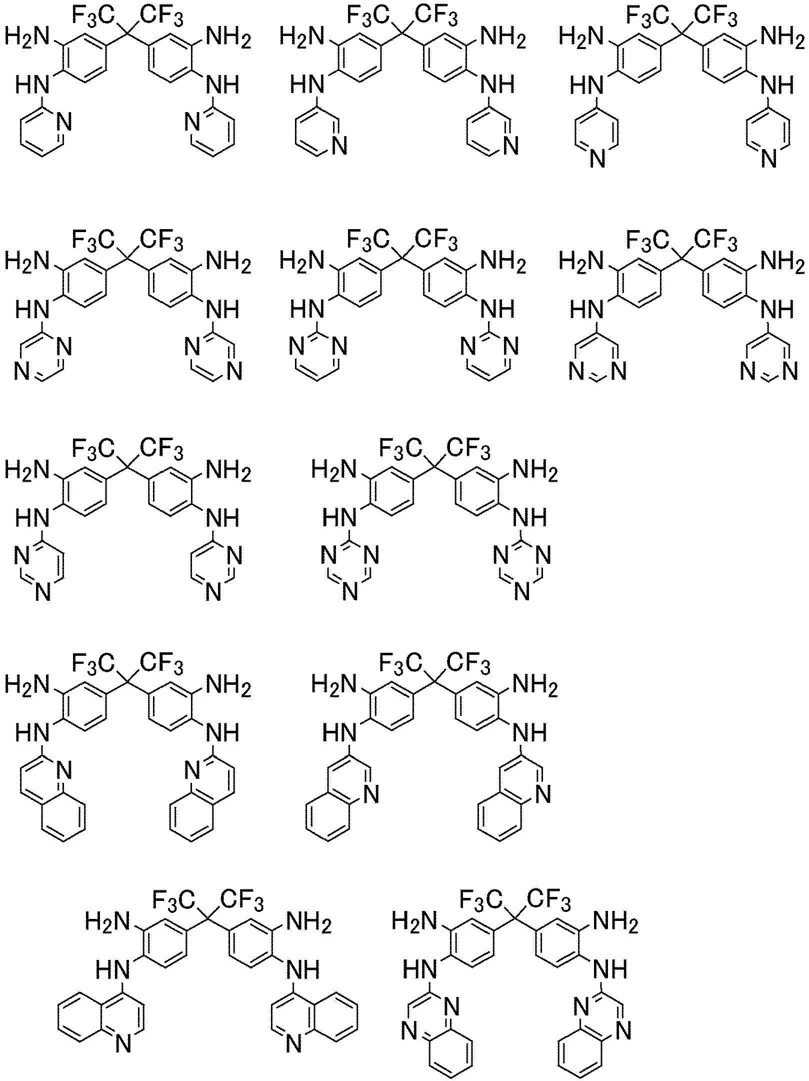

Preferred examples of the compound of the disclosure also include compounds represented by the following formulas. In each formula, R represents an alkyl group, preferably a C1-C5 alkyl group, more preferably a methyl group (-CH₃).

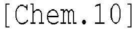

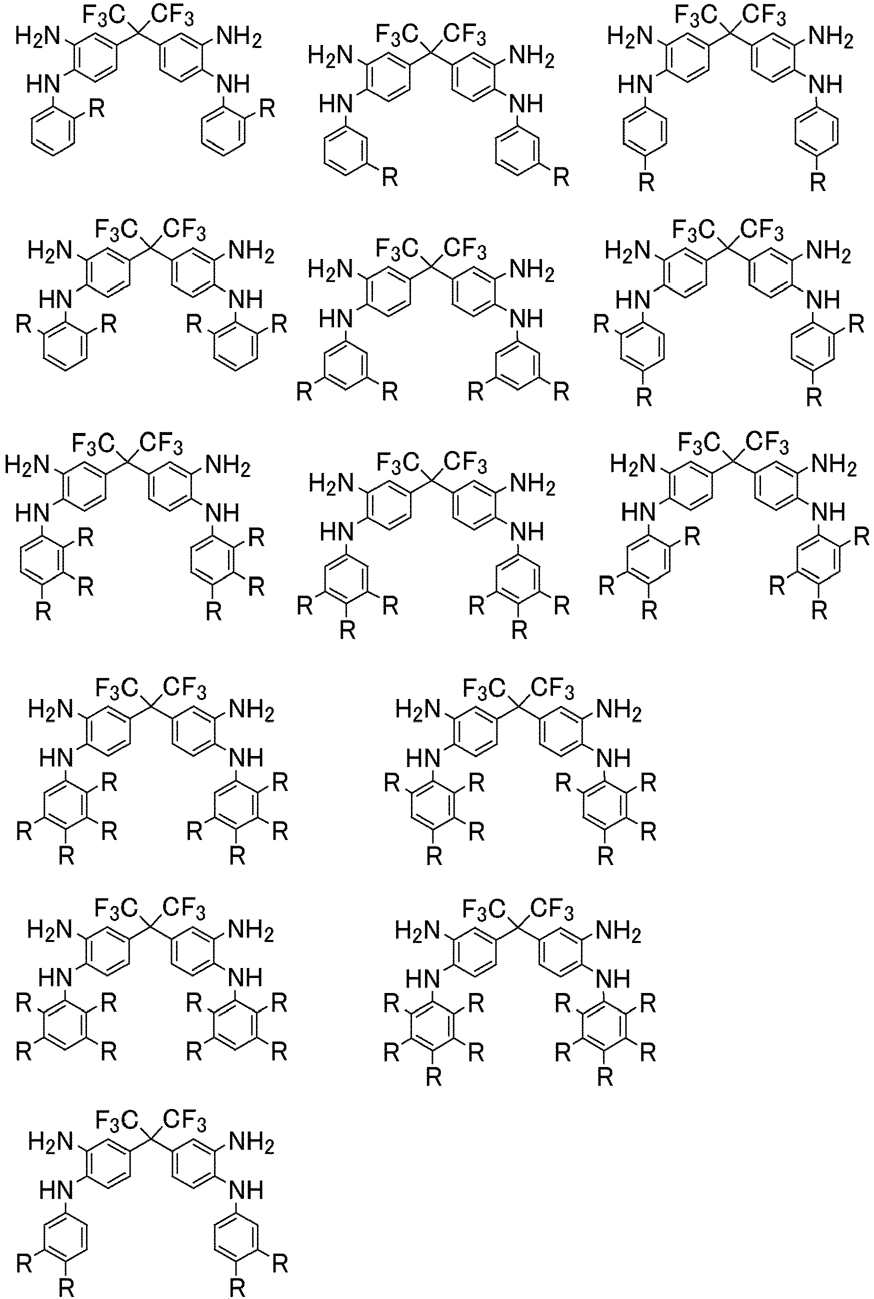

In order to provide a cross-linked product with even better properties in terms of heat resistance, plasma resistance, compression set properties, and crack resistance, the compound of the disclosure is preferably a compound represented by any of the following formulas.

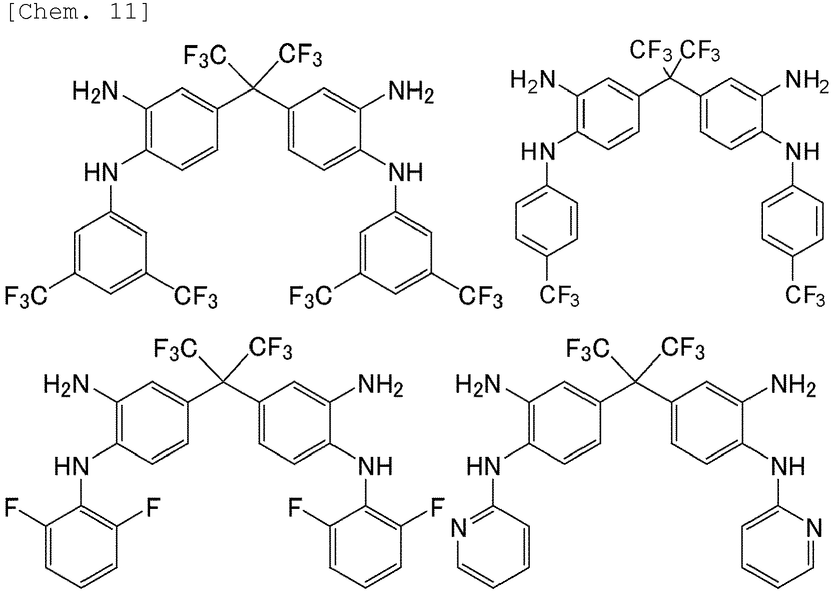

In order to promote cross-linking, preferred is a compound represented by the following formula.

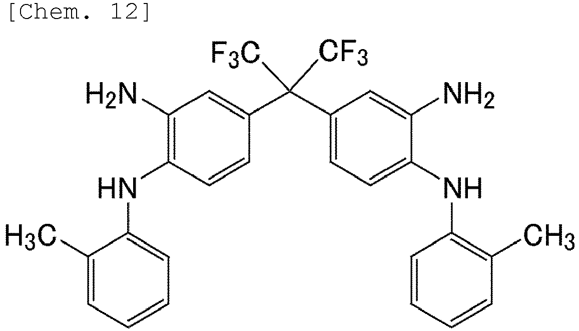

The compound preferably has a melting point of 100°C or higher, more preferably 120°C or higher, still more preferably 130°C or higher. The melting point is preferably 300°C or lower, more preferably 280°C or lower, still more preferably 260°C or lower.

The melting point within the above range can industrially advantageously reduce contamination of the mold during molding.

The melting point is a temperature corresponding to the maximum value on a heat-of-fusion curve with a temperature-increasing rate of 10°C/min using a thermogravimetric/differential thermal analyzer [TG-DTA].

The compound can be suitably produced by a production method including:
(1) nitrating a compound (100) represented by the following formula (100): 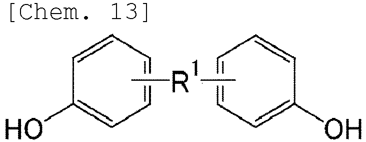 (wherein R¹ is as defined above) to obtain a compound (101) represented by the following formula (101): 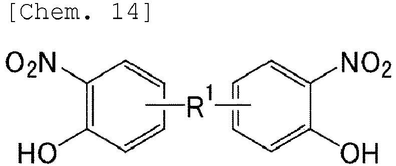 (wherein R¹ is as defined above);
(2) sulfonylating the compound (101) to obtain a compound (102) represented by the following formula (102): 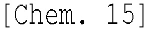 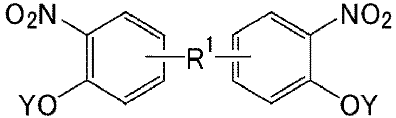 (wherein R¹ is as defined above; and Y is a group represented by -S(=O)₂-R² where R² is a hydrocarbon group optionally containing a substituent);
(3) reacting the compound (102) with a compound (103) represented by the following formula (103): 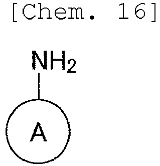 (wherein A is as defined above) to obtain a compound (104) represented by the following formula (104): 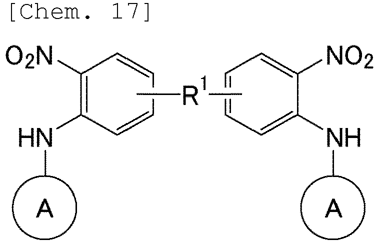 (wherein R¹ and A are as defined above); and
(4) reducing the compound (104) to obtain a compound represented by the formula (1).

The substituent optionally contained in the hydrocarbon group for R² in Y in the formula (102) is preferably a halogen atom, a nitro group, or an alkyl group, more preferably a fluorine atom or a nitro group.

R² is preferably an alkyl group optionally containing a substituent or a phenyl group optionally containing a substituent, more preferably a methyl group, a trifluoromethyl group, a tolyl group, or a nitrophenyl group.

Nitration in the step (1) can be carried out by reacting the compound (100) with a nitrating agent such as nitric acid, and conventionally known reaction conditions can be employed.

Sulfonylation in the step (2) can be carried out by reacting the compound (101) with a sulfonylating agent. The sulfonylating agent is preferably a compound that reacts with the hydroxy group of the compound (101) to give a leaving group represented by -O-S(=O)₂-R² (wherein R² is as defined above). Examples thereof include tosyl anhydride, tosyl chloride, tosyl bromide, trifluoromethanesulfonic anhydride, trifluoromethanesulfonic chloride, trifluoromethanesulfonic bromide, McMurry reagent, Comins reagent, methanesulfonic chloride, methanesulfonic bromide, methanesulfonic anhydride, nitrobenzenesulfonic chloride, nitrobenzenesulfonic bromide, and nitrobenzenesulfonic anhydride. Preferred among these are tosyl chloride, trifluoromethanesulfonic anhydride, trifluoromethanesulfonic chloride, and trifluoromethanesulfonic bromide. In order to allow more efficient elimination of the leaving group in the step (3), more preferred are trifluoromethanesulfonic anhydride, trifluoromethanesulfonic chloride, and trifluoromethanesulfonic bromide, and particularly preferred are trifluoromethanesulfonic anhydride and trifluoromethanesulfonic chloride.

In the sulfonylation, the sulfonylating agent is preferably used in an amount of 0.8 to 10 mol, more preferably 1.0 to 5 mol, per 1 mol of the compound (101).

The sulfonylation is preferably carried out in the presence of a base. Examples of the base include amines and inorganic bases.

Examples of amines include triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,8-diazabicyclo[5.4.0]-7-undecene (DBU), 1,5-diazabicyclo[4.3.0]-5-nonene, 1,4-diazabicyclo[2.2.2]octane (DABCO), 4-dimethylaminopyridine (DMAP), and proton sponge.

Examples of inorganic bases include lithium hydroxide, potassium hydroxide, sodium hydroxide, calcium hydroxide, lithium carbonate, sodium carbonate, potassium carbonate, sodium hydrogen carbonate, potassium hydrogen carbonate, cesium carbonate, cesium hydrogen carbonate, lithium hydrogen carbonate, cesium fluoride, potassium fluoride, sodium fluoride, lithium chloride, and lithium bromide.

Among these, the base is preferably an amine, more preferably triethylamine, tributylamine, 4-dimethylaminopyridine (DMAP), or pyridine.

The base is preferably used in an amount of 0.001 to 7 mol, more preferably 0.1 to 3 mol, per 1 mol of the compound (101).

The sulfonylation can be carried out in a solvent. The solvent is preferably an organic solvent. Examples thereof include: non-aromatic hydrocarbon solvents such as pentane, hexane, heptane, octane, cyclohexane, decahydronaphthalene, n-decane, isododecane, and tridecane; aromatic hydrocarbon solvents such as benzene, toluene, xylene, tetralin, veratrol, diethylbenzene, methylnaphthalene, nitrobenzene, o-nitrotoluene, mesitylene, indene, and diphenyl sulfide; ketones such as acetone, methyl ethyl ketone, methyl isobutyl ketone, acetophenone, propiophenone, diisobutyl ketone, and isophorone; halogenated hydrocarbon solvents such as dichloromethane, chloroform, and chlorobenzene; ether solvents such as diethyl ether, tetrahydrofuran, diisopropyl ether, methyl t-butyl ether, dioxane, dimethoxyethane, diglyme, phenetole, 1,1-dimethoxycyclohexane, and diisoamyl ether; ester solvents such as ethyl acetate, isopropyl acetate, diethyl malonate, 3-methoxy-3-methylbutyl acetate, γ-butyrolactone, ethylene carbonate, propylene carbonate, dimethyl carbonate, and α-acetyl-γ-butyrolactone; nitrile solvents such as acetonitrile and benzonitrile; sulfoxide solvents such as dimethylsulfoxide and sulfolane; and amide solvents such as N,N-dimethylformamide, N,N-dimethylacetamide, N-methylpyrrolidone, 1,3-dimethyl-2-imidazolidinone, N,N-dimethylacrylamide, N,N-dimethylacetoacetamide, N,N-diethylformamide, and N,N-diethylacetamide.

Preferred among these are benzene, toluene, xylene, dichloromethane, and chloroform.

The sulfonylation temperature is preferably -20°C to 80°C, more preferably 0°C to 50°C.

The sulfonylation pressure is preferably 0.05 to 0.20 MPa, more preferably 0.07 to 0.15 MPa.

The sulfonylation time is preferably 0.5 to 24 hours, more preferably 1 to 15 hours.

In the reaction in the step (3), the compound (103) is preferably used in an amount of 1.0 to 20 mol, more preferably 1.0 to 5.0 mol, per 1 mol of the compound (102).

The reaction in the step (3) is preferably carried out in the presence of a palladium catalyst. Any palladium catalyst may be used. Preferably, an organic palladium complex is used.

Examples of the palladium catalyst include a zero-valent palladium complex; a zero-valent palladium complex generated from a divalent palladium complex during the reaction; and a complex obtainable by mixing any of the foregoing complexes with at least one compound (ligand) selected from the group consisting of diketones, phosphines, diamines, and bipyridyls.

Any zero-valent palladium complex may be used. Examples thereof include Pd₂(DBA)₃ (DBA is dibenzylideneacetone), Pd(COD)₂ (COD is cycloocta-1,5-diene), Pd(DPPE) (DPPE is 1,2-bisdiphenylphosphinoethane), Pd(PCy₃)₂ (Cy is a cyclohexyl group), Pd(Pt-Bu₃)₂ (t-Bu is a t-butyl group), and Pd(PPh₃)₄ (Ph is a phenyl group).

Examples of divalent palladium complexes include palladium chloride, palladium bromide, palladium acetate, bis(acetylacetonato)palladium (II), dichloro(η4-1,5-cyclooctadiene)palladium (II), and a complex in which a phosphine ligand such as triphenylphosphine is coordinated to any of the foregoing complexes.

Examples of complexes in which phosphine ligands are coordinated include dichloro[1,2-bis(diphenylphosphino)ethane]palladium (II),
dichloro[1,3-bis(diphenylphosphino)propane]palladium (II),
dichloro[1,4-bis(diphenylphosphino)butane]palladium (II),
dichloro[1,5-bis(diphenylphosphino)pentane]palladium (II),
dichloro[bis(diphenylphosphinophenyl)ether]palladium (II),
dichloro[bis(dicyclohexylphosphinophenyl)ether] palladium (II),
dichloro[4,5-bis(diphenylphosphino)-9,9'-dimethylxanthene]palladium (II),
dichloro[1,1'-bis(diphenylphosphino)ferrocene]palladium (II),
dichloro[1,1'-bis(di-tert-butylphosphino)ferrocene]palladium (II),
dichloro[1,1'-bis(dicyclohexylphosphino)ferrocene]palladium (II),
dichloro[1,1'-bis(diisopropylphosphino)ferrocene]palladium (II),
dichloro[2,2'-bis(diphenylphosphino)-1,1'-binaphthyl]palladium (II),
dichloro[4,6-bis(diphenylphosphino)phenoxazine]palladium (II),
dichloro[1,3-bis(diisopropylphosphino)propane]palladium (II),
dichloro[1,4-bis(diisopropylphosphino)butane]palladium (II),
dichloro[1,3-bis(dicyclohexylphosphino)propane]palladium (II), and
dichloro[1,4-bis(dicyclohexylphosphino)butane]palladium (II).

These divalent palladium complexes are, for example, reduced by coexisting reducing species (e.g., phosphine, zinc, organometallic reagents) during the reaction to produce zero-valent palladium complexes.

The zero-valent palladium complex or the zero-valent palladium complex generated from any of the divalent palladium complexes by reduction can interact with a compound (ligand) added during the reaction as needed, such as a diketone, phosphine, diamine, or bipyridyl, to be converted into a zero-valent palladium complex that participates in the reaction. It should be noted that it is not necessarily clear how many of these ligands are coordinated to the zero-valent palladium complex during the reaction.

These palladium complexes are often used in the reaction in the form of uniform solutions with reaction substrates by using the ligands as described above. Alternatively, they can be dispersed or supported in/on polymers such as polystyrene or polyethylene and used as heterogeneous catalysts. Such heterogeneous catalysts are advantageous in the reaction process in terms of catalyst recovery or the like. An example of a specific catalyst structure is a structure represented by the following chemical formula, in which a metal atom is fixed using, for example, a polymer phosphine in which phosphine is introduced into a cross-linked polystyrene (PS) chain. In addition to this, polymer phosphines disclosed in the following documents are also usable:
1) Kanbara et al., Macromolecules, 2000, vol. 33, p. 657;
2) Yamamoto et al., J. Polym. Sci., 2002, vol. 40, p. 2637;
3) JP H06-32763 A;
4) JP 2005-281454 A; and
5) JP 2009-527352 A.

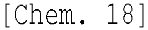

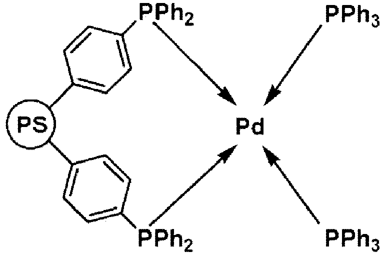

In the formula, PS is polystyrene and Ph is a phenyl group.

The diketone may be, for example, a β-diketone such as acetylacetone, 1-phenyl-1,3-butanedione, or 1,3-diphenylpropanedione.

The phosphine may be, for example, a phosphine including one or more substituents selected from the group consisting of an optionally substituted alkyl group, an optionally substituted cycloalkyl group, and an optionally substituted aryl group, on the phosphorus atom. Particularly preferred is a tri(cyclo)alkylphosphine, a triarylphosphine, or a Buchwald ligand. Specific examples of tri(cyclo)alkylphosphines include tri(C3-20(cyclo)alkyl)phosphines such as tricyclohexylphosphine, triisopropylphosphine, tri-t-butylphosphine, trithexylphosphine, triadamantylphosphine, tricyclopentylphosphine, di-t-butylmethylphosphine, tribicyclo[2,2,2]octylphosphine, and trinorbornylphosphine. Specific examples of triarylphosphines include tri(monocyclic aryl)phosphines such as triphenylphosphine, trimesitylphosphine, and tri(o-tolyl)phosphine. Examples of Buchwald ligands include 2-dicyclohexylphosphino-2'-(N,N-dimethylamino)biphenyl, 2-dicyclohexylphosphino-2',4',6'-triisopropylbiphenyl, 2-dicyclohexylphosphino-2',6'-dimethoxybiphenyl, 2-di-tert-butylphosphino-2',4',6'-triisopropylbiphenyl, (2-biphenyl)dicyclohexylphosphine, (2-biphenyl)di-tert-butylphosphine, 2-dicyclohexylphosphino-2',6'-diisopropoxybiphenyl, 2-di-tert-butylphosphino-3,4,5,6-tetramethyl-2',4',6'-triisopropyl-1,1'-biphenyl, 2'-(dicyclohexylphosphino)-2,6-dimethoxy-sodium salt (sSPhos), 2-di-tert-butylphosphino-2'-methylbiphenyl, 2-dicyclohexylphosphino-2'-methylbiphenyl, and 2-diphenylphosphino-2'-(N,N-dimethylamino) biphenyl.

Preferred among these are triphenylphosphine, tricyclohexylphosphine, and tri-t-butylphosphine. Also effective are 2-dicyclohexylphosphino-2'4'6'-triisopropylbiphenyl and [4-(N,N-dimethylamino)phenyl]di-tert-butylphosphine; and bidentate ligands such as 4,5-bis(diphenylphosphino)-9,9-dimethyl-9H-xanthene, 1,4-bis(diphenylphosphino)butane, 1,3-bis(diphenylphosphino)propane, 1,1'-bis(diphenylphosphino)ferrocene and 2,2'-bis(diphenylphosphino)-1,1'-binaphthyl.

As described above, arylphosphines for heterogeneous catalysts in which phosphine units are introduced into polymer chains can also be preferably used. A specific example thereof is triarylphosphine in which one phenyl group of triphenylphosphine binds to the polymer chain, as shown in the chemical formula below.

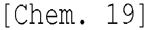

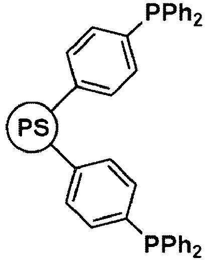

In the formula, PS is polystyrene and Ph is a phenyl group.

The diamine may be, for example, tetramethylethylenediamine or 1,2-diphenylethylenediamine.

Among these ligands, preferred are phosphines, diamines, and bipyridyls, more preferred are triarylphosphines, and particularly preferred is triphenylphosphine.

The palladium catalyst is preferably used in an amount of 0.002 to 40 mol, more preferably 0.02 to 30 mol, still more preferably 0.1 to 20 mol, particularly preferably 1 to 20 mol, per 1 mol of the compound (102).

The reaction in the step (3) is preferably carried out in the presence of a base. Examples of the base include amines, inorganic bases, and organometallic bases.

Examples of amines include triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,8-diazabicyclo[5.4.0]-7-undecene (DBU), 1,5-diazabicyclo[4.3.0]-5-nonene, 1,4-diazabicyclo[2.2.2]octane (DABCO), 4-dimethylaminopyridine (DMAP), and proton sponge.

Examples of inorganic bases include lithium hydroxide, potassium hydroxide, sodium hydroxide, calcium hydroxide, lithium carbonate, sodium carbonate, potassium carbonate, sodium hydrogen carbonate, potassium hydrogen carbonate, cesium carbonate, cesium hydrogen carbonate, lithium hydrogen carbonate, cesium fluoride, potassium fluoride, sodium fluoride, lithium chloride, and lithium bromide.

Examples of organometallic bases include organic alkali metal compounds such as butyllithium, t-butyllithium, phenyllithium, triphenylmethylsodium, and ethylsodium; organic alkaline earth metal compounds such as methylmagnesium bromide, dimethylmagnesium, phenylmagnesium chloride, phenylcalcium bromide, and bis(dicyclopentadiene)calcium; and alkoxides such as sodium methoxide and t-butyl methoxide.

Among these, the base is preferably cesium carbonate, cesium hydrogen carbonate, cesium fluoride, triethylamine, tri(n-propyl)amine, tri(n-butyl)amine, pyridine, lutidine, γ-collidine, or 1,8-diazabicyclo[5.4.0]-7-undecene (DBU).

The base is preferably used in an amount of 0.5 to 10 mol, more preferably 1 to 6 mol, per 1 mol of the compound (102) .

The reaction in the step (3) can be carried out in a solvent. The solvent is preferably an organic solvent. Examples thereof include: non-aromatic hydrocarbon solvents such as pentane, hexane, heptane, octane, cyclohexane, decahydronaphthalene, n-decane, isododecane, and tridecane; aromatic hydrocarbon solvents such as benzene, toluene, xylene, tetralin, veratrol, diethylbenzene, methylnaphthalene, nitrobenzene, o-nitrotoluene, mesitylene, indene, and diphenyl sulfide; ketones such as acetone, methyl ethyl ketone, methyl isobutyl ketone, acetophenone, propiophenone, diisobutyl ketone, and isophorone; halogenated hydrocarbon solvents such as dichloromethane, chloroform, and chlorobenzene; ether solvents such as diethyl ether, tetrahydrofuran (THF), diisopropyl ether, methyl t-butyl ether, dioxane, dimethoxyethane, diglyme, phenetole, 1,1-dimethoxycyclohexane, and diisoamyl ether; ester solvents such as butyl acetate, ethyl acetate, isopropyl acetate, diethyl malonate, 3-methoxy-3-methylbutyl acetate, γ-butyrolactone, ethylene carbonate, propylene carbonate, dimethyl carbonate, and α-acetyl-γ-butyrolactone; nitrile solvents such as acetonitrile and benzonitrile; sulfoxide solvents such as dimethylsulfoxide and sulfolane; and amide solvents such as N,N-dimethylformamide, N,N-dimethylacetamide, N-methylpyrrolidone, 1,3-dimethyl-2-imidazolidinone, N,N-dimethylacrylamide, N,N-dimethylacetoacetamide, N,N-diethylformamide, and N,N-diethylacetamide.

Preferred among these are benzene, toluene, tetrahydrofuran, dioxane, dimethoxyethane, diglyme, butyl acetate, N,N-dimethylformamide, N,N-dimethylacetamide, N-methylpyrrolidone, and 1,3-dimethyl-2-imidazolidinone.

The reaction temperature in the step (3) is preferably 20°C to 150°C, more preferably 50°C to 130°C.

The reaction pressure in the step (3) is preferably 0.05 to 0.20 MPa, more preferably 0.07 to 0.15 MPa.

The reaction time in the step (3) is preferably 0.5 to 48 hours, more preferably 1 to 24 hours.

The reduction in the step (4) can be carried out by bringing the compound (104) into contact with a reducing agent such as hydrogen in the presence of a palladium catalyst such as palladium carbon, and conventionally known reaction conditions can be employed.

After completion of each step, the resulting product may be separated and purified by distilling off the solvent, column chromatography, recrystallization, or the like.

The compound of the disclosure can be suitably used as a cross-linking agent for providing a cross-linked product with excellent plasma resistance (in particular, oxygen plasma resistance) while maintaining heat resistance. It can also be suitably used as a cross-linking agent for providing a cross-linked product with good compression set properties and good crack resistance.

When the compound of the disclosure is used as a cross-linking agent, the moiety represented by the following formula: 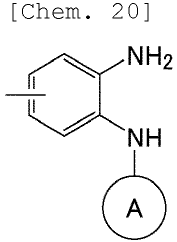 in the formula (1) functions as a cross-linkable reactive group.

The compound of the disclosure can also be suitably used as a monomer for super engineering plastics.

A cross-linking agent containing the compound of the disclosure is also an aspect of the present disclosure. The cross-linking agent is preferably a cross-linking agent for fluorine-containing elastomers. The cross-linking agent is preferably a cross-linking agent for imidazole cross-linking.

The disclosure also relates to a composition containing a fluorine-containing elastomer and the compound of the disclosure or the cross-linking agent of the disclosure described above. Cross-linking the composition of the disclosure can provide a cross-linked product with excellent plasma resistance (in particular, oxygen plasma resistance) while maintaining heat resistance. Also, a cross-linked product with good compression set properties and good crack resistance can be obtained.

The fluorine-containing elastomer may be a partially fluorinated elastomer or a perfluoroelastomer. A perfluoroelastomer is preferably used because it has better chemical resistance and better heat resistance.

Examples of partially fluorinated elastomers include a vinylidene fluoride (VdF)-based fluororubber, a tetrafluoroethylene (TFE)/propylene (Pr)-based fluororubber, a tetrafluoroethylene (TFE)/propylene/vinylidene fluoride (VdF)-based fluororubber, an ethylene/hexafluoropropylene (HFP)-based fluororubber, an ethylene/hexafluoropropylene (HFP)/vinylidene fluoride (VdF)-based fluororubber, and an ethylene/hexafluoropropylene (HFP)/tetrafluoroethylene (TFE)-based fluororubber. Preferred among these is at least one selected from the group consisting of vinylidene fluoride-based fluororubbers and tetrafluoroethylene/propylene-based fluororubbers.

The vinylidene fluoride-based fluororubber is preferably a copolymer containing 45 to 85 mol% of a polymerized unit based on vinylidene fluoride and 55 to 15 mol% of a polymerized unit based on at least one different monomer copolymerizable with vinylidene fluoride, preferably a copolymer containing 50 to 80 mol% of a polymerized unit based on vinylidene fluoride and 50 to 20 mol% of a polymerized unit based on at least one different monomer copolymerizable with vinylidene fluoride.

The amounts of the monomers constituting the fluorine-containing elastomer herein can be calculated by any appropriate combination of NMR, FT-IR, elemental analysis, and X-ray fluorescence analysis in accordance with the types of the monomers.

Examples of the at least one different monomer copolymerizable with vinylidene fluoride include monomers such as tetrafluoroethylene (TFE), hexafluoropropylene (HFP), fluoroalkyl vinyl ether, chlorotrifluoroethylene (CTFE), trifluoroethylene, trifluoropropylene, pentafluoropropylene, trifluorobutene, tetrafluoroisobutene, hexafluoroisobutene, vinyl fluoride, a fluoromonomer represented by the formula (6): CH₂=CFRf⁶¹ (wherein Rf⁶¹ is a C1-C12 linear or branched fluoroalkyl group)), a fluoromonomer represented by the formula (7): CH₂=CH-(CF₂)ₙ-X² (wherein X² is H or F; and n is an integer of 3 to 10), and a monomer that gives a cross-linking site; and non-fluorinated monomers such as ethylene, propylene, and alkyl vinyl ether. These may be used alone or in any combination. Preferred among these is at least one selected from the group consisting of TFE, HFP, fluoroalkyl vinyl ether, and CTFE.

The fluoroalkyl vinyl ether preferably includes at least one selected from the group consisting of:
a fluoromonomer represented by the formula (8):

   CF₂=CF-ORf⁸¹

   (wherein Rf⁸¹ is a C1-C8 perfluoroalkyl group),
a fluoromonomer represented by the formula (10): CF₂=CFOCF₂ORf¹⁰¹
   (wherein Rf¹⁰¹ is a C1-C6 linear or branched perfluoroalkyl group, a C5-C6 cyclic perfluoroalkyl group, or a C2-C6 linear or branched perfluorooxyalkyl group containing 1 to 3 oxygen atoms), and
a fluoromonomer represented by the formula (11): CF₂=CFO(CF₂CF(Y¹¹)O)ₘ(CF₂)ₙF
   (wherein Y¹¹ is a fluorine atom or a trifluoromethyl group; m is an integer of 1 to 4; and n is an integer of 1 to 4). More preferred are fluoromonomers represented by the formula (8).

Specific examples of vinylidene fluoride-based fluororubbers include a VdF/HFP-based rubber, a VdF/HFP/TFE-based rubber, a VdF/CTFE-based rubber, a VdF/CTFE/TFE-based rubber, a VdF/fluoromonomer represented by the formula (6)-based rubber, a VdF/fluoromonomer represented by the formula (6)/TFE-based rubber, a VdF/perfluoro(methyl vinyl ether) (PMVE)-based rubber, a VdF/PMVE/TFE-based rubber, and a VdF/PMVE/TFE/HFP-based rubber. The VdF/fluoromonomer represented by the formula (6)-based rubber is preferably a VdF/CH₂=CFCF₃-based rubber, and the VdF/fluoromonomer represented by the formula (6)/TFE-based rubber is preferably a VdF/TFE/CH₂=CFCF₃-based rubber.

The VdF/CH₂=CFCF₃-based rubber is preferably a copolymer containing 40 to 99.5 mol% of a polymerized unit based on VdF and 0.5 to 60 mol% of a polymerized unit based on CH₂=CFCF₃, more preferably a copolymer containing 50 to 85 mol% of a polymerized unit based on VdF and 20 to 50 mol% of a polymerized unit based on CH₂=CFCF₃.

The tetrafluoroethylene/propylene-based fluororubber preferably contains 45 to 70 mol% of a polymerized unit based on tetrafluoroethylene, 55 to 30 mol% of a polymerized unit based on propylene, and 0 to 5 mol% of a polymerized unit based on a fluoromonomer that gives a cross-linking site.

The fluorine-containing elastomer may be a perfluoroelastomer. The perfluoroelastomer is preferably a perfluoroelastomer containing a polymerized unit based on TFE. For example, the perfluoroelastomer preferably includes at least one selected from the group consisting of a TFE/fluoromonomer represented by the formula (8), (10), or (11) copolymer and a TFE/fluoromonomer represented by the formula (8), (10), or (11)/monomer that gives a cross-linking site copolymer.

In the case of a TFE/PMVE copolymer, the composition thereof is preferably (45 to 90)/(10 to 55) (mol%), more preferably (55 to 80)/(20 to 45), still more preferably (55 to 70) / (30 to 45).

In the case of a TFE/PMVE/monomer that gives a cross-linking site copolymer, the composition thereof is preferably (45 to 89.9)/(10 to 54.9)/(0.01 to 4) (mol%), more preferably (55 to 77.9)/(20 to 49.9)/(0.1 to 3.5), still more preferably (55 to 69.8)/(30 to 44.8)/(0.2 to 3).

In the case of a TFE/C4-C12 fluoromonomer represented by the formula (8), (10), or (11) copolymer, the composition thereof is preferably (50 to 90)/(10 to 50) (mol%), more preferably (60 to 88)/(12 to 40), still more preferably (65 to 85)/(15 to 35).

In the case of a TFE/C4-C12 fluoromonomer represented by the formula (8), (10), or (11)/monomer that gives a cross-linking site copolymer, the composition thereof is preferably (50 to 89.9)/(10 to 49.9)/(0.01 to 4) (mol%), more preferably (60 to 87.9)/(12 to 39.9)/(0.1 to 3.5), still more preferably (65 to 84.8)/(15 to 34.8)/(0.2 to 3).

When the composition is out of this range, the properties of a rubber elastic body tend to be lost and the properties tend to become closer to those of resin.

The perfluoroelastomer preferably includes at least one selected from the group consisting of a TFE/fluoromonomer represented by the formula (11)/fluoromonomer that gives a cross-linking site copolymer, a TFE/perfluorovinyl ether represented by the formula (11) copolymer, a TFE/fluoromonomer represented by the formula (8) copolymer, and a TFE/fluoromonomer represented by the formula (8)/monomer that gives a cross-linking site copolymer.

Examples of the perfluoroelastomer also include perfluoroelastomers disclosed in documents such as WO 97/24381 A, JP S61-57324 B, JP H04-81608 B, and JP H05-13961 B.

The "monomer that gives a cross-linking site" refers to a monomer (cure site monomer) containing a cross-linkable group that provides the fluoropolymer with a cross-linking site for forming a cross-link with a cross-linking agent.

The monomer that gives a cross-linking site preferably includes at least one selected from the group consisting of:
fluoromonomers represented by the formula (12):

   CX³₂=CX³-R_{f}¹²¹CHR¹²¹X⁴

   (wherein X³ is a hydrogen atom, a fluorine atom, or CH₃; R_{f}¹²¹ is a fluoroalkylene group, a perfluoroalkylene group, a fluoro(poly)oxyalkylene group, or a
   perfluoro(poly)oxyalkylene group; R¹²¹ is a hydrogen atom or CH₃; and X⁴ is an iodine atom or a bromine atom);
fluoromonomers represented by the formula (13): CX³₂=CX³-R_{f}¹³¹X⁴
   (wherein, X³ is a hydrogen atom, a fluorine atom, or CH₃; R_{f}¹³¹ is a fluoroalkylene group, a perfluoroalkylene group, a fluoropolyoxyalkylene group, or a perfluoropolyoxyalkylene group; and X⁴ is an iodine atom or a bromine atom);
fluoromonomers represented by the formula (14): CF₂=CFO(CF₂CF(CF₃)O)ₘ(CF₂)ₙ-X⁵
   (wherein m is an integer of 0 to 5; n is an integer of 1 to 3; and X⁵ is a cyano group, a carboxyl group, an alkoxycarbonyl group, an iodine atom, a bromine atom, or - CH₂I) ;
fluoromonomers represented by the formula (15): CH₂=CFCF₂O(CF(CF₃)CF₂O)ₘ(CF(CF₃))ₙ-X⁶
   (wherein m is an integer of 0 to 5; n is an integer of 1 to 3; and X⁶ is a cyano group, a carboxyl group, an alkoxycarbonyl group, an iodine atom, a bromine atom, or - CH₂OH); and
monomers represented by the formula (16): CR¹⁶²R¹⁶³=CR¹⁶⁴-Z-CR¹⁶⁵=CR¹⁶⁶R¹⁶⁷

(wherein R¹⁶², R¹⁶³, R¹⁶⁴, R¹⁶⁵, R¹⁶⁶, and R¹⁶⁷ are the same as or different from each other, and are each a hydrogen atom or a C1-C5 alkyl group; Z is a linear or branched group optionally having an oxygen atom and is a C1-C18 alkylene group, a C3-C18 cycloalkylene group, a C1-C10 alkylene or oxyalkylene group that is at least partially fluorinated, or a (per)fluoropolyoxyalkylene group that has a molecular weight of 500 to 10000 and is represented by the formula: - (Q)ₚ-CF₂O-(CF₂CF₂O)ₘ(CF₂O)ₙ-CF₂-(Q)ₚ-
wherein Q is an alkylene group or an oxyalkylene group; p is 0 or 1; and m/n is 0.2 to 5.

X³ is preferably a fluorine atom. R_{f}¹²¹ and R_{f}¹³¹ are preferably C1-C5 perfluoroalkylene groups. R¹²¹ is preferably a hydrogen atom. X⁵ is preferably a cyano group, an alkoxycarbonyl group, an iodine atom, a bromine atom, or -CH₂I. X⁶ is preferably a cyano group, an alkoxycarbonyl group, an iodine atom, a bromine atom, or - CH₂OH.

The monomer that gives a cross-linking site preferably includes at least one selected from the group consisting of CF₂=CFOCF₂CF(CF₃)OCF₂CF₂CN, CF₂=CFOCF₂CF(CF₃)OCF₂CF₂COOH, CF₂=CFOCF₂CF(CF₃)OCF₂CF₂CH₂I, CF₂=CFOCF₂CF₂CH₂I, CH₂=CFCF₂OCF(CF₃)CF₂OCF(CF₃)CN, CH₂=CFCF₂OCF(CF₃)CF₂OCF(CF₃)COOH, CH₂=CFCF₂OCF(CF₃)CF₂OCF(CF₃)CH₂OH, CH₂=CHCF₂CF₂I, CH₂=CH(CF₂)₂CH=CH₂, CH2=CH(CF₂)₆CH=CH₂, and CF₂=CFO(CF₂)₅CN, more preferably at least one selected from the group consisting of CF₂=CFOCF₂CF(CF₃)OCF₂CF₂CN and CF₂=CFOCF₂CF₂CH₂I.

In order to give excellent compression set properties at high temperature, the fluorine-containing elastomer preferably has a glass transition temperature of -70°C or higher, more preferably -60°C or higher, still more preferably -50°C or higher. In order to give good cold resistance, the glass transition temperature is preferably 5°C or lower, more preferably 0°C or lower, still more preferably -3°C or lower.

The glass transition temperature can be determined as follows. Specifically, using a differential scanning calorimeter (DSC822e, available from Mettler-Toledo International Inc.), 10 mg of a sample is heated at 20°C/min to give a DSC curve, and the temperature is read at the intermediate point of two intersections between each of the extension lines of the base lines before and after the secondary transition of the DSC curve and the tangent line at the inflection point of the DSC curve.

In order to give good heat resistance, the fluorine-containing elastomer preferably has a Mooney viscosity ML(1+20) at 170°C of 30 or higher, more preferably 40 or higher, still more preferably 50 or higher. In order to give good processibility, the Mooney viscosity is preferably 150 or lower, more preferably 120 or lower, still more preferably 110 or lower.

In order to give good heat resistance, the fluorine-containing elastomer preferably has a Mooney viscosity ML(1+20) at 140°C of 30 or higher, more preferably 40 or higher, still more preferably 50 or higher. In order to give good processibility, the Mooney viscosity is preferably 180 or lower, more preferably 150 or lower, still more preferably 110 or lower.

In order to give good heat resistance, the fluorine-containing elastomer preferably has a Mooney viscosity ML(1+10) at 100°C of 10 or higher, more preferably 20 or higher, still more preferably 30 or higher. In order to give good processibility, the Mooney viscosity is preferably 120 or lower, more preferably 100 or lower, still more preferably 80 or lower.

The Mooney viscosity can be determined using a Mooney viscometer MV2000E available from Alpha Technologies Inc. at 170°C, 140°C, or 100°C in conformity with JIS K 6300.

The partially fluorinated elastomer and perfluoroelastomer described above can be produced by conventional methods. An iodine compound or a bromine compound can be used as a chain transfer agent because the polymer to be obtained has a narrow molecular weight distribution, which facilitates control of the molecular weight, and an iodine or bromine atom can be introduced to an end. A polymerization method in which an iodine or bromine compound is used includes, for example, emulsion polymerization in an aqueous medium under pressure in the presence of an iodine or bromine compound in a substantially oxygen-free state (iodine transfer polymerization method). A typical example of the iodine or bromine compound to be used is a compound represented by the following formula:

R¹³IₓBr_{y}

wherein, x and y are each an integer of 0 to 2 and satisfy 1 ≤ x + y ≤ 2; and R¹³ is a C1-C16 saturated or unsaturated fluorohydrocarbon or chlorofluorohydrocarbon group, or a C1-C3 hydrocarbon group optionally containing an oxygen atom. Use of an iodine or bromine compound introduces an iodine or bromine atom into the polymer and the introduced atom functions as a cross-linking point.

Examples of the iodine compound and the bromine compound include 1,3-diiodoperfluoropropane, 2-iodoperfluoropropane, 1,3-diiodo-2-chloroperfluoropropane, 1,4-diiodoperfluorobutane, 1,5-diiodo-2,4-dichloroperfluoropentane, 1,6-diiodoperfluorohexane, 1,8-diiodoperfluorooctane, 1,12-diiodoperfluorododecane, 1,16-diiodoperfluorohexadecane, diiodomethane, 1,2-diiodoethane, 1,3-diiodo-n-propane, CF₂Br₂, BrCF₂CF₂Br, CF₃CFBrCF₂Br, CFClBr₂, BrCF₂CFClBr, CFBrClCFClBr, BrCF₂CF₂CF₂Br, BrCF₂CFBrOCF₃ , 1-bromo-2-iodoperfluoroethane, 1-bromo-3-iodoperfluoropropane, 1-bromo-4-iodoperfluorobutane, 2-bromo-3-iodoperfluorobutane, 3-bromo-4-iodoperfluorobutene-1,2-bromo-4-iodoperfluorobutene-1, monoiodomonobromo-substituted benzene, diiodomonobromo-substituted benzene, and (2-iodoethyl)-substituted benzene and (2-bromoethyl)-substituted benzene. These compounds may be used alone or in combination with each other.

From the viewpoints of properties such as polymerization reactivity, cross-linking reactivity, and easy availability, preferably used among these are 1,4-diiodoperfluorobutane, 1,6-diiodoperfluorohexane, and 2-iodoperfluoropropane.

The fluorine-containing elastomer preferably contains a cross-linkable group capable of reacting with the cross-linkable reactive group of the compound of the disclosure. The cross-linkable group preferably includes at least one selected from the group consisting of a hydroxy group (-OH), a cyano group (-CN), a carboxyl group (-COOH), an alkoxycarbonyl group, and an acid halide group, more preferably at least one selected from the group consisting of a cyano group, a carboxyl group, an alkoxycarbonyl group, and an acid halide group, still more preferably at least one selected from the group consisting of a cyano group and a carboxyl group.

When the fluorine-containing elastomer contains the cross-linkable group, it can react with the cross-linking agent of the disclosure, and a cross-linked product with even better properties in terms of heat resistance, plasma resistance, compression set properties, and crack resistance can be obtained.

The fluorine-containing elastomer may have the cross-linkable group in the main chain and/or a side chain.

The fluorine-containing elastomer is preferably a fluorine-containing elastomer containing a cyano group (-CN group) in the main chain and/or a side chain. The cyano group in the fluorine-containing elastomer containing a cyano group (-CN group) in the main chain and/or a side chain reacts with the amino group of the compound or the cross-linking agent to form an imidazole ring, whereby the fluorine-containing elastomer can be cross-linked. The fluorine-containing elastomer can impart excellent compression set and heat resistance to molded articles.

Examples of the fluorine-containing elastomer containing a cyano group (-CN group) in the main chain and/or a side chain include perfluoroelastomers and non-perfluoroelastomers. The "perfluoroelastomer" herein refers to one containing a perfuoromonomer unit in an amount of 90 mol% or more of all the structural units.

A perfluoroelastomer containing a cyano group (-CN group) in the main chain and/or a side chain may be, for example, a perfluoroelastomer containing tetrafluoroethylene/a perfluoro(alkyl vinyl ether)/a monomer containing a cyano group (-CN group). The composition of tetrafluoroethylene/a perfluoro(alkyl vinyl ether) is preferably (50 to 90)/(10 to 50) (mol%), more preferably (50 to 80)/(20 to 50) (mol%), still more preferably (55 to 75)/(25 to 45) (mol%). A monomer containing a cyano group (-CN group) is preferably contained in an amount of 0.1 to 5 mol%, more preferably 0.3 to 3 mol%, relative to the total amount of tetrafluoroethylene and a perfluoro(alkyl vinyl ether) from the viewpoint of good cross-linking properties and good heat resistance.

Examples of the perfluoro(alkyl vinyl ether) in this case include perfluoro(methyl vinyl ether) and perfluoro(propyl vinyl ether). These can be used alone or in any combination.

Examples of the cyano group (-CN group)-containing monomer include monomers represented by any of the following formulas (31) to (47):

CY¹₂=CY¹(CF₂)ₙ-X¹ (31),

wherein Y¹ is a hydrogen atom or a fluorine atom; and n is an integer of 1 to 8;

CF₂=CFCF₂R_{f}²-X¹ (32),

wherein R_{f}² is -(OCF₂)ₙ- or -(OCF(CF₃))ₙ-; and n is an integer of 0 to 5;

CF₂=CFCF₂(OCF(CF₃)CF₂)ₘ(OCH₂CF₂CF₂)ₙOCH₂CF₂-X¹ (33),

wherein m is an integer of 0 to 5; and n is an integer of 0 to 5;

CF₂=CFCF₂(OCH₂CF₂CF₂)ₘ(OCF(CF₃)CF₂)ₙOCF(CF₃)-X¹ (34),

wherein m is an integer of 0 to 5; and n is an integer of 0 to 5;

CF₂=CF(OCF₂CF(CF₃))ₘO(CF₂)ₙ-X¹ (35),

wherein m is an integer of 0 to 5; and n is an integer of 1 to 8;

CF₂=CF(OCF₂CF(CF₃))m-X¹ (36),

wherein m is an integer of 1 to 5;

CF₂=CFOCF₂(CF(CF₃)OCF₂)ₙCF(-X¹)CF₃ (37),

wherein n is an integer of 1 to 4;

CF₂=CFO(CF₂)ₙOCF(CF₃)-X¹ (38),

wherein n is an integer of 2 to 5;

CF₂=CFO(CF₂)ₙ-(C₆H₄)-X¹ (39),

wherein n is an integer of 1 to 6;

CF₂=CF(OCF₂CF(CF₃))ₙOCF₂CF(CF₃)-X¹ (40),

wherein n is an integer of 1 or 2;

CH₂=CFCF₂O(CF(CF₃)CF₂O)ₙCF(CF₃)-X¹ (41),

wherein n is an integer of 0 to 5;

CF₂=CFO(CF₂CF(CF₃)O)ₘ(CF₂)ₙ-X¹ (42),

wherein m is an integer of 0 to 5; and n is an integer of 1 to 3;

CH₂=CFCF₂OCF(CF₃)OCF(CF₃)-X¹ (43) ;

CH₂=CFCF₂OCH₂CF₂-X¹ (44);

CF₂=CFO(CF₂CF(CF₃)O)ₘCF₂CF(CF₃)-X¹ (45),

wherein m is an integer of 0 or greater;

CF₂=CFOCF(CF₃)CF₂O(CF₂)ₙ-X¹ (46),

wherein n is an integer of 1 or greater; and

CF₂=CFOCF₂OCF₂CF(CF₃)OCF₂-X¹ (47) .

In the formulas (31) to (47), X¹ is a cyano group (-CN group). These monomers may be used alone or in any combination.

Preferred among these is a monomer represented by the formula (35) or (42), and more preferred is CF₂=CFOCF₂CF(CF₃)OCF₂CF₂CN.

These perfluoroelastomers can be produced by conventional methods.

Specific examples of such perfluoroelastomers include fluororubbers described in documents such as WO 97/24381 A, JP S61-57324 B, JP H04-81608 B, and JP H05-13961 B.

Examples of the non-perfluoroelastomer containing a cyano group (-CN group) in the main chain and/or a side chain include a vinylidene fluoride (VdF)-based fluororubber, a tetrafluoroethylene (TFE)/propylene-based fluororubber, a tetrafluoroethylene

(TFE)/propylene/vinylidene fluoride (VdF)-based fluororubber, an ethylene/hexafluoroethylene (HFP)-based fluororubber, an ethylene/hexafluoropropylene (HFP)/vinylidene fluoride (VdF)-based fluororubber, an ethylene/hexafluoropropylene (HFP)/tetrafluoroethylene (TFE)-based fluororubber, a fluorosilicone-based fluororubber, and a fluorophosphazene-based fluororubber. These may be used alone or in any combination as long as the effects of the invention are not impaired.

The vinylidene fluoride-based fluororubber refers to a fluorine-containing copolymer containing 45 to 85 mol% of a polymerized unit based on vinylidene fluoride and 55 to 15 mol% of a polymerized unit based on at least one different monomer copolymerizable with vinylidene fluoride, preferably, a fluorine-containing copolymer containing 50 to 80 mol% of a polymerized unit based on vinylidene fluoride and 50 to 20 mol% of a polymerized unit based on at least one different monomer copolymerizable with vinylidene fluoride.

Examples of the at least one different monomer copolymerizable with vinylidene fluoride include fluorine-containing monomers such as tetrafluoroethylene (TFE), chlorotrifluoroethylene (CTFE), trifluoroethylene, hexafluoropropylene (HFP), trifluoropropylene, tetrafluoropropylene, pentafluoropropylene, trifluorobutene, tetrafluoroisobutene, perfluoro(alkyl vinyl ether) (PAVE), and vinyl fluoride and nonfluorine-containing monomers such as ethylene, propylene, and alkyl vinyl ether. These may be used alone or may be used in any combination. Preferred among these are tetrafluoroethylene, hexafluoropropylene, and perfluoro(alkyl vinyl ether).

Specific examples of rubber include VdF-HFP-based rubbers, VdF-HFP-TFE-based rubbers, VdF-CTFE-based rubbers, and VdF-CTFE-TFE-based rubbers.

A tetrafluoroethylene/propylene-based fluororubber refers to a fluorine-containing copolymer containing 45 to 70 mol% of a polymerized unit based on tetrafluoroethylene and 55 to 30 mol% of a polymerized unit based on propylene, and further containing a polymerized unit based on a monomer that gives a cross-linking site in an amount of 0 to 5 mol% relative to the total amount of the polymerized unit based on tetrafluoroethylene and the polymerized unit based on propylene.

Examples of monomers that give cross-linking sites include cyano group-containing monomers such as those disclosed in JP H04-505345 T and JP H05-500070 T, and the monomers represented by the above-mentioned formulas (31) to (47).

These non-perfluoroelastomers can be produced by conventional methods.

The fluorine-containing elastomer used may be a thermoplastic fluororubber containing an elastomeric fluorine-containing polymer chain segment and a non-elastomeric fluoropolymer chain segment, or a rubber composition containing the fluororubber and a thermoplastic fluororubber.

In order to simplify the process, the acid treatment is preferably applied as a coagulation technique for isolating the polymerization product from the polymerization reaction mixture. Also, the polymerization mixture may be subjected to acid treatment and then the polymer product may be isolated by lyophilization, for example. Furthermore, a method of coagulation by ultrasonic waves or mechanical power may be employed.

The cyano group may be introduced by the method disclosed in WO 00/05959 A.

The fluorine-containing elastomer preferably has a low metal content. The fluorine-containing elastomer having a low metal content is preferable because it provides a molded article that can be used in the semiconductor production process or pharmaceutical production process in which contamination by metal components must be avoided.

The metal content in the fluorine-containing elastomer is preferably 100 ppm or less, more preferably 50 ppm or less, still more preferably 10 ppm or less.

The metal content can be measured by flameless atomic absorption spectrometry or inductively coupled plasma optical emission spectrometry. The metal content herein refers to the total metal content of Fe, Cr, Ni, Cu, Al, Na, Mg, Ca, Zn, Ba, and K.

The amount of the cross-linking agent of the disclosure is preferably 0.05 to 10 parts by mass, more preferably 0.5 to 5 parts by mass, per 100 parts by mass of the fluorine-containing elastomer. Less than 0.05 parts by mass of the cross-linking agent tends to cause insufficient cross-linking of the fluorine-containing polymer, while more than 10 parts by mass thereof tends to impair the physical properties of the cross-linked product.

One type of the cross-linking agent of the disclosure may be used alone or two or more types thereof may be used in combination.

The cross-linking agent of the disclosure can be used in combination with a different compound. For example, the cross-linking agent of the disclosure may be used in combination with at least one selected from the group consisting of inorganic nitrides, organotin compounds, ammonia-generating compounds, and cross-linking agents (excluding the cross-linking agent of the disclosure). Use of any of these in combination can promote cross-linking.

Examples of inorganic nitrides include, but are not limited to, silicon nitride (Si₃N₄), lithium nitride, titanium nitride, aluminum nitride, boron nitride, vanadium nitride, and zirconium nitride. In order to give nanometer-scale fine particles, preferred among these is silicon nitride.

Examples of organotin compounds include tetraphenyltin and triphenyltin.

A preferred ammonia-generating compound is a compound that generates ammonia at 40°C to 330°C.

The ammonia-generating compound is preferably urea or a derivative thereof or an ammonium salt, more preferably urea or an ammonium salt, still more preferably urea. The ammonium salt may be an organic ammonium salt or an inorganic ammonium salt. The ammonia-generating compound may be a compound that reacts with a trace amount of water to generate ammonia.

Examples of derivatives of urea include biurea, thiourea, urea hydrochloride, and biuret.

Examples of organic ammonium salts include the compounds disclosed in JP H09-111081 A, WO 00/09603 A, and WO 98/23675 A, including ammonium salts of polyfluorocarboxylic acids such as ammonium perfluorohexanoate and ammonium perfluorooctanoate; ammonium salts of polyfluorosulfonic acids such as ammonium perfluorohexanesulfonate and ammonium perfluorooctanesulfonate; ammonium salts of polyfluoroalkyl group-containing phosphoric acid or phosphonic acid such as ammonium perfluorohexanephosphate and ammonium perfluorooctanephosphate; and ammonium salts of non-fluorinated carboxylic acids or sulfonic acids such as ammonium benzoate, ammonium adipate, and ammonium phthalate.

Examples of inorganic ammonium salts include the compounds disclosed in JP H09-111081 A, such as ammonium sulfate, ammonium carbonate, ammonium nitrate, and ammonium phosphate.

Examples of ammonia-generating compounds also include acetaldehyde ammonia, hexamethylenetetramine, formamidine, formamidine hydrochloride, formamidine acetate, t-butylcarbamate, benzylcarbamate, HCF₂CF₂CH(CH₃)OCONH₂, and phthalamide.

Examples of the cross-linking agent (other than the cross-linking agent of the disclosure) include cross-linking agents to be used in peroxide cross-linking, polyol cross-linking, polyamine cross-linking, triazine cross-linking, oxazole cross-linking, imidazole cross-linking, and thiazole cross-linking. In the case where the fluorine-containing elastomer contains a cyano group (-CN group), the cross-linking agent preferably includes at least one selected from the group consisting of oxazole cross-linking agents, imidazole cross-linking agents, and thiazole cross-linking agents.

The cross-linking agent used in peroxide cross-linking is an organic peroxide that can easily generate a peroxy radical in the presence of heat or a redox system. Examples thereof include 2,5-dimethyl-2,5-di(t-butylperoxy) hexane. Usually, the type and amount of the organic peroxide are selected in consideration of the amount of active -O-O- and the decomposition temperature.

The cross-linking aid that can be used in this case is a compound having reactivity with a peroxy radical and a polymer radical. Examples thereof include multifunctional compounds containing a functional group such as -CH=CH₂, - CH₂CH=CH₂, -CF=CF₂, -C(CF₃)CF₂, -C(CH₃)=CF₂, -CF=CF(CF₃), - CF=CF(CH₃), -C(C₆H₅)=CF₂, -CF=CF(C₆H₅), -CH=CF₂, -CF=CHF, - C(CF₃)=CHF, -CF=CH(CF₃), and -CH=CF(CF₃) ("C₆H₅" in each formula represents a phenyl group). Specific examples thereof include triallyl cyanurate, triallyl isocyanurate (TAIC), triacrylformal, triallyl trimellitate, N,N'-n-phenylene bismaleimide, dipropargyl terephthalate, diallyl phthalate, tetraallyl terephthalate amide, triallyl phosphate, bismaleimide, fluorinated triallyl isocyanurate (1,3,5-tris(2,3,3-trifluoro-2-propenyl)-1,3,5-triazine-2,4,6-trione), tris(diallylamine)-S-triazine, triallyl phosphite, N,N-diallyl acrylamide, and 1,6-divinyl dodecafluorohexane.

The cross-linking aid used together with the peroxide cross-linking agent may be a compound represented by the following formula (48): 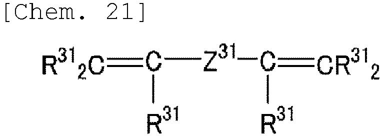 (wherein six R³¹s are each independently H, a halogen atom, or a C1-C5 optionally halogenated group into which an ether bond is optionally inserted; and Z³¹ is a C1-C18 optionally halogenated linear or branched alkylene, cycloalkylene, or (per)fluoropolyoxyalkylene group optionally containing a heteroatom).

Examples of the compound represented by the formula (48) include: a compound represented by the following formula (49): 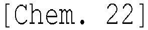 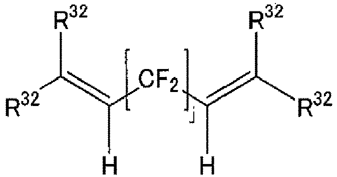 (wherein, j is an integer of 2 to 10, preferably 4 to 8; and four R³²s are each independently H, F, or a C1-C5 alkyl or (per)fluoroalkyl group); a compound represented by the following formula (50): 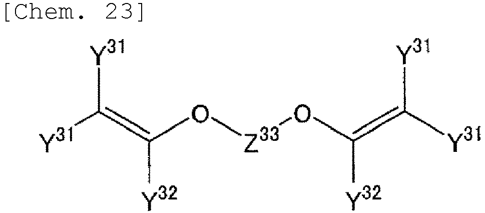 (wherein Y³¹s are each independently F, Cl or H; Y³²s are each independently F, Cl, H or OR³³ (wherein R³³ is a branched or linear alkyl group that may be partially, substantially, or completely fluorinated or chlorinated); and Z³³ is an optionally fluorinated, C2-C10 divalent group into which an ether bond is optionally inserted; Z³³ is preferably a group represented by -(CF₂)ₘ- wherein m is an integer of 3 to 5; and the compound represented by the formula (50) is preferably F₂C=CF-O-(CF₂)₅-O-CF=CF₂); and a compound represented by the following formula (51): 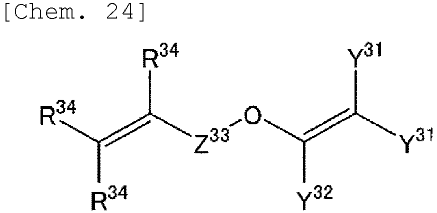 (wherein Y³¹, Y³², and Z³³ are defined as above: and R³⁴s are each independently H, F, or a C1-C5 alkyl or (per)fluoroalkyl group).

Examples of a cross-linking aid to be used together with the cross-linking agent or the peroxide cross-linking agent include a compound containing at least one structure represented by the following formula (52): 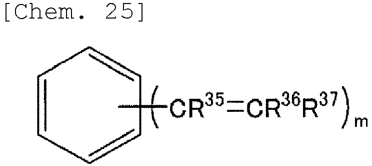 (wherein R³⁵ to R³⁷ are each independently a hydrogen atom, a fluorine atom, an alkyl group, a fluorinated alkyl group, or a substituted or non-substituted aryl group, at least one selected from R³⁵ to R³⁷ is a fluorine atom or a group containing a fluorine atom; m is an integer of 1 to 5; when m is 2 or greater, the m R³⁵s to R³⁷s may be the same as or different from each other; and hydrogen atom(s) in the benzene ring may be replaced). When m is 1, the compound preferably contains two or more of the structures.

Examples of the compound containing a structure represented by the formula (52) include a compound represented by the following formula (53): 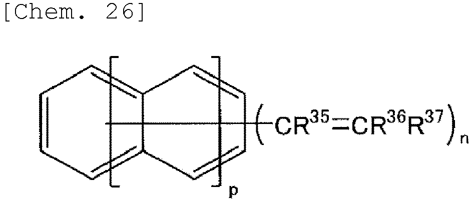 (wherein R³⁵ to R³⁷ are as defined above; p is an integer of 0 to 2; and n is an integer of 2 to 6): and a compound represented by the following formula (54): 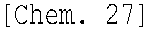 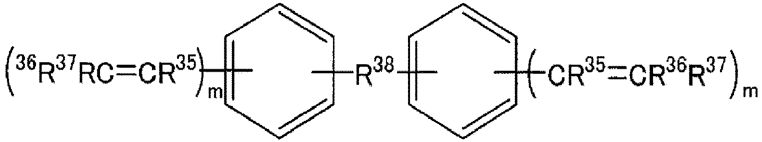 (wherein R³⁵ to R³⁷ are as defined above; R³⁸ is a single bond, -SO₂-, -O-, -S-, -CO-, a hetero atom-containing group, a substituted or non-substituted alkylene group, a substituted or non-substituted cycloalkylene group, or a substituted or non-substituted arylene group; m is an integer of 1 to 5; and each of these groups may be partially or completely fluorinated).

Any hetero atom-containing group that is a divalent group containing a hetero atom may be used. Examples of hetero atoms include an oxygen atom, a nitrogen atom, a sulfur atom, a boron atom, and a phosphorus atom.

Examples of the cross-linking agent used in polyol cross-linking include polyhydric alcohol compounds such as bisphenol A and bisphenol AF.

Examples of the cross-linking agent used in polyamine cross-linking include polyamine compounds such as hexamethylenediamine carbamate, N,N'-dicinnamylidene-1,6-hexanediamine, and 4,4'-bis(aminocyclohexyl)methanecarbamate.

Examples of cross-linking agents used in oxazole cross-linking, imidazole cross-linking, and thiazole cross-linking include bisdiaminophenyl-based cross-linking agents, bisaminophenol-based cross-linking agents, and bisaminothiophenol-based cross-linking agents represented by the following formula (55): 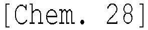 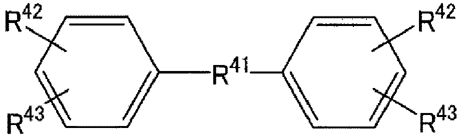 (wherein R⁴¹ is -SO₂-, -O-, -CO-, a C1-C6 alkylene group, a C1-C10 perfluoroalkylene group, or a single bond, or a group represented by the following formula: 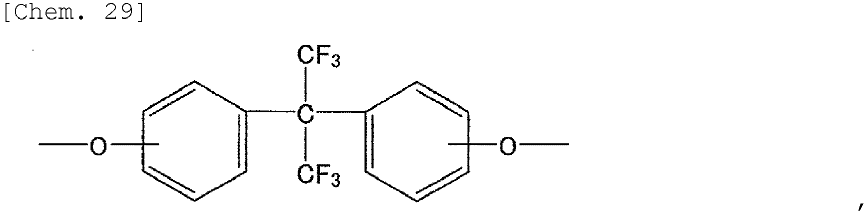 one of R⁴² or R⁴³ is -NH₂ and the other is -NHR⁴⁴, -NH₂, -OH, or -SH; and R⁴⁴ is a hydrogen atom, a fluorine atom, or a monovalent organic group; preferably R⁴² is -NH₂ and R⁴³ is -NHR⁴⁴).

Preferred specific examples of the C1-C6 alkylene group include methylene, ethylene, propylene, butylene, pentylene, and hexylene groups. An example of the C1-C10 perfluoroalkylene group is a group represented by the following formula:

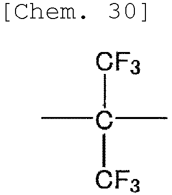

These compounds are known as examples of bisdiaminophenyl compounds from JP H02-59177 B and JP H08-120146 A.

Examples of cross-linking agents used in oxazole cross-linking, imidazole cross-linking, and thiazole cross-linking also include bisamidrazone-based cross-linking agents represented by the following formula (56): 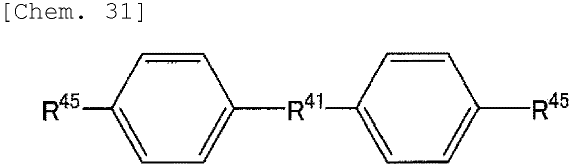 (wherein R⁴¹ is as defined above; and R⁴⁵s are each independently any of the groups represented by the following formulas: 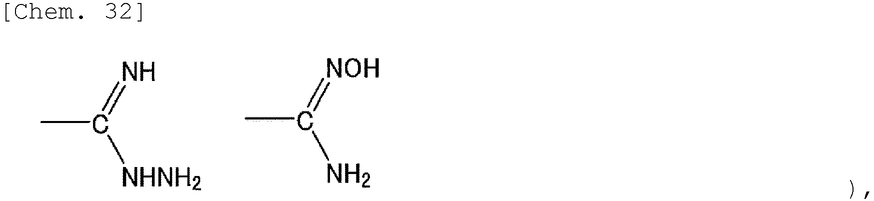 amidrazone-based cross-linking agents represented by the following formula (57): 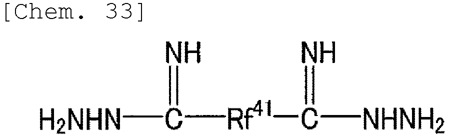 (wherein Rf⁴¹ is a C1-C10 perfluoroalkylene group), bisamidoxime-based cross-linking agents represented by the following formula (58): 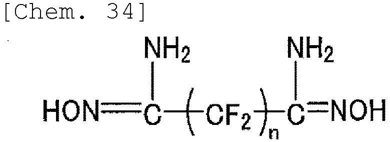
(wherein n is an integer of 1 to 10), a compound represented by the formula (59): HN=CR⁴⁵R⁴⁶
(wherein R⁴⁵ is selected from the group consisting of H, NH₂, and NHR⁴⁷; and R⁴⁶ is selected from the group consisting of Ph, SO₂H, NR⁴⁸R⁴⁹, 2-pyridine, and CH₂CONH₂, where R⁴⁷ is selected from the group consisting of Ph, NH₂, and CN; R⁴⁸ is selected from the group consisting of H, NHPh, CH₂CONH₂, a C1-C8 linear alkyl group, and a C1-C8 branched alkyl group; and R⁴⁹ is selected from the group consisting of Ph, COOC(CH₃)₃, NH₂, CH₂COOH, CSNH₂, CNHNH₃⁺Cl⁻, p-phenyl CN, 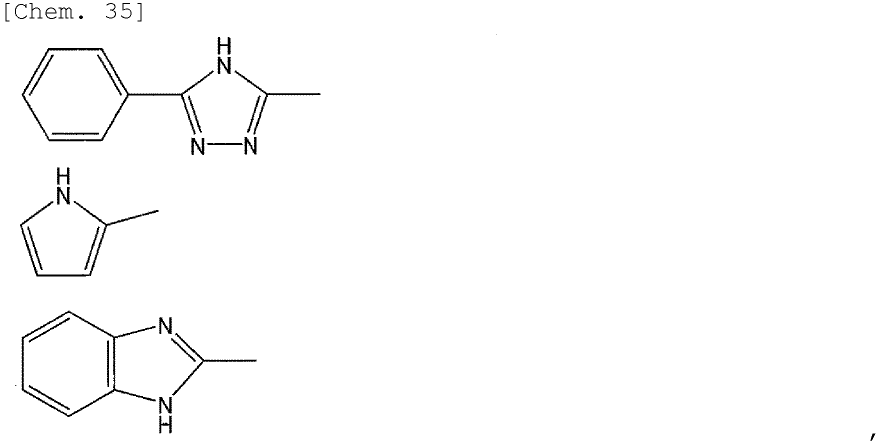
and COPh).

These bisaminophenol-based cross-linking agents, bisaminothiophenol-based cross-linking agents, bisdiaminophenyl-based cross-linking agents have been conventionally used in a cross-linking system in which the cross-linking site is a cyano group, and also react with a carboxyl group and an alkoxycarbonyl group and form an oxazole ring, a thiazole ring, and an imidazole ring, respectively, to give a cross-linked product.

Examples of the cross-linking agents further include cross-linking agents represented by the formula (60): X⁴¹-(CH₂)ₙ-R⁵⁰-(CH₂)ₘ-X⁴¹ (wherein X⁴¹s are each independently an alkyne group, a nitrile group, or Y⁴¹_{P}N₃ (where Y⁴¹ is SO, SO₂, C₆H₄, or CO; and p is 0 or 1); n and m are each independently an integer of 1 to 4; and R⁵⁰ is selected from the group consisting of:
i) a C3-C10 fluoroalkylene group,
ii) a C3-C10 fluoroalkoxylylene group,
iii) a substituted arylene group,
iv) an oligomer containing a copolymerized unit of vinylidene fluoride and perfluoro(methyl vinyl ether),
v) an oligomer containing a copolymerized unit of vinylidene fluoride and hexafluoropropylene,
vi) an oligomer containing a copolymerized unit of tetrafluoroethylene and perfluoro(methyl vinyl ether), and
vii) an oligomer containing a copolymerized unit of tetrafluoroethylene and a hydrocarbon olefin). The cross-linking agent is preferably used together with a fluorine-containing elastomer containing a nitrile, azide, sulfonylazide, carbonylazide, or alkyne group. For example, the nitrile group of the fluorine-containing elastomer reacts with the azide group of the cross-linking agent to form a tetrazole ring, whereby a cross-linked product is provided.

The cross-linking agent is particularly preferably a compound containing multiple 3-amino-4-hydroxyphenyl or 3-amino-4-mercaptophenyl groups, or a compound represented by the following formula (61): 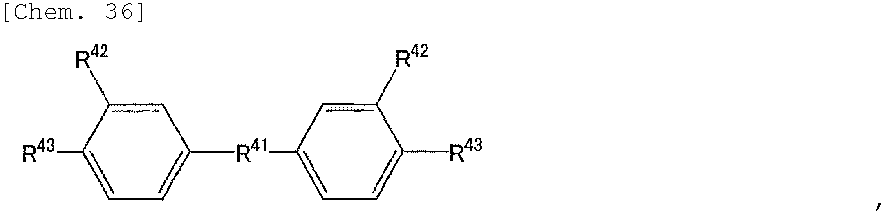 wherein R⁴¹, R⁴² and R⁴³ are as defined above. Specific examples include 2,2-bis(3-amino-4-hydroxyphenyl)hexafluoropropane (common name: bis(aminophenol) AF), 2,2-bis(3-amino-4-mercaptophenyl)hexafluoropropane, tetraaminobenzene, bis-3,4-diaminophenylmethane, bis-3,4-diaminophenyl ether, 2,2-bis(3,4-diaminophenyl)hexafluoropropane, 2,2-bis[3-amino-4-(N-phenylamino)phenyl]hexafluoropropane, 2,2-bis[3 -amino-4-(N-methylamino)phenyl]hexafluoropropane, 2,2-bis[3-amino-4-(N-ethylamino)phenyl]hexafluoropropane, 2,2-bis[3-amino-4-(N-propylamino)phenyl]hexafluoropropane, 2,2-bis[3-amino-4-(N-perfluorophenylamino)phenyl]hexafluoropropane, and 2,2-bis[3-amino-4-(N-benzylamino)phenyl]hexafluoropropane.

Preferred among these as the cross-linking agent is 2,2-bis[3-amino-4-(N-phenylamino)phenyl]hexafluoropropane from the viewpoint of heat resistance, steam resistance, amine resistance, and good cross-linking properties.

The amount of the at least one selected from the group consisting of inorganic nitrides, organotin compounds, ammonia-generating compounds, and cross-linking agents (excluding the cross-linking agent of the disclosure) is preferably 0.01 to 10 parts by mass, more preferably 0.05 to 5 parts by mass, per 100 parts by mass of the fluorine-containing elastomer.

The composition may contain common fillers.

Examples of common fillers include: imide-based fillers having an imide structure such as polyimide, polyamideimide, and polyetherimide; organic fillers made of engineering plastics such as polyarylate, polysulfone, polyether sulfone, polyhenylene sulfide, polyether ether ketone, polyether ketone, and polyoxybenzoate; metal oxide fillers such as aluminum oxide, silicon oxide, and yttrium oxide; metal carbides such as silicon carbide and aluminum carbide; metal nitride fillers such as silicon nitride and aluminum nitride; and inorganic fillers such as aluminum fluoride, fluorocarbon, and carbon black.

Preferred among these are aluminum oxide, yttrium oxide, silicon oxide, polyimide, fluorocarbon, silicon carbide, silicon nitride, and aluminum nitride from the viewpoint of shielding effect against various plasmas.

One type of the inorganic filler or organic filler may be compounded alone, or two or more types thereof may be compounded in combination.

The amount of the common fillers is preferably 0.5 to 100 parts by mass, more preferably 5 to 50 parts by mass, per 100 parts by mass of the fluorine-containing elastomer.

Especially in fields where high purity and non-contamination properties are not required, to the composition may be added common additives conventionally compounded to fluorine-containing polymer compositions as needed, such as fillers, processing aids, plasticizers, and colorants. One or more commonly used cross-linking agents or cross-linking aids different from those described above may be compounded.

The composition can be produced by kneading the compound of the disclosure or the cross-linking agent of the disclosure with the fluorine-containing elastomer.

The kneading can be performed using a common processing machine for polymers, such as an open roll mill, a Banbury mixer, a kneader, or a closed mixer.

The composition can be suitably used as a molding material for obtaining a cross-linked product through cross-linking molding.

The disclosure also relates to a molded article obtainable by cross-linking the composition of the disclosure. The molded article of the disclosure has excellent plasma resistance while maintaining heat resistance.

The molded article of the disclosure can be produced by molding the composition of the disclosure and cross-linking the resulting molded article, or by performing molding and cross-linking simultaneously. A coating film can also be obtained by applying the cross-linkable composition and cross-linking the applied composition.

Any molding method may be employed such as compression molding, extrusion molding, transfer molding, or injection molding.

The cross-linking conditions are preferably determined according to the type of the cross-linking agent used. For example, cross-linking is preferably carried out at a temperature of 140°C to 300°C for 1 minute to 24 hours. Cross-linking can be performed under normal pressure, increased pressure, or reduced pressure, or in the air.

The cross-linking method may be, but is not limited to, steam cross-linking, pressure molding, or a usual method in which the cross-linking reaction is initiated by heating. It may be radiation cross-linking at normal temperature and normal pressure.

Only the initial cross-linking treatment (primary cross-linking) may be carried out. Alternatively, posttreatment referred to as secondary cross-linking may be carried out after the primary cross-linking.

When the primary cross-linking and the secondary cross-linking are carried out, the primary cross-linking is preferably carried out at 150°C to 250°C for 5 to 120 minutes, more preferably at 170°C to 210°C for 5 to 60 minutes. The cross-linking means used may be a known cross-linking means, such as press cross-linking.

The secondary cross-linking is preferably carried out at 180°C to 320°C for 2 to 24 hours, more preferably at 280°C to 310°C for 5 to 20 hours. STEP cross-linking may be used. The cross-linking means used may be a known cross-linking means, such as oven cross-linking.

The molded article of the disclosure has excellent heat resistance, and is generally used for parts intended to slide in contact with other materials, seal or enclose other materials and substances, and for vibration and sound insulation. It can be used as various parts in various fields such as the automobile industry, the aircraft industry, and the semiconductor industry.

Examples of the fields where the molded article is used include the field relating to semiconductors, the field of automobiles, the field of aircraft, the field of space and rockets, the field of shipping, the field of chemistry such as chemical plants, the field of chemicals such as pharmaceuticals, the field of photography such as film processors, the field of printing such as printers, the field of coating such as coating equipment, the field of analysis and physical and chemical instruments such as analyzers and meters, the field of food machinery such as food plant equipment and household items, the field of beverage and food production equipment, the field of drug production equipment, the field of medical parts, the field of equipment for transporting chemicals, the field of equipment for nuclear power plants, the field of steel such as sheet steel processing equipment, the field of general industry, the field of electrics, the field of fuel cells, the field of electronic parts, the field of parts of optical devices, the field of parts of space devices, the field of equipment for petrochemical plants, the field of parts of equipment for prospecting and mining energy sources such as petroleum and gas, the field of oil refining, and the field of parts of equipment for transporting petroleum.

The molded article of the disclosure may be used in any of various forms such as seal materials and packings, including rings, packings, gaskets, diaphragms, oil seals, bearing seals, lip seals, plunger seals, door seals, lip and face seals, gas delivery plate seals, wafer support seals, and barrel seals. The seal materials may be used in applications requiring heat resistance, solvent resistance, chemical resistance, and non-stickiness.

The molded article of the disclosure may also be used as any of tubes, hoses, rolls, rubber rolls, flexible joints, rubber plates, coatings, belts, dampers, valves, valve sheets, valve bodies, chemical-resistant coating materials, laminating materials, and lining materials.

The cross-sectional shapes of the above rings, packings, and seals may be any various shapes, such as a rectangular shape, an O-like shape, and a ferrule shape, and any deformed shapes such as D-like, L-like, T-like, V-like, X-like, and Y-like shapes.

In the field relating to semiconductors, the molded article of the disclosure may be used for semiconductor manufacturing devices, liquid crystal panel manufacturing devices, plasma panel manufacturing devices, plasma display panel manufacturing devices, plasma addressed liquid crystal panel manufacturing devices, organic EL panel manufacturing devices, field emission display panel manufacturing devices, solar cell substrate manufacturing devices, and semiconductor transporting devices. Examples of such devices include CVD devices, gas control devices such as gas control devices for semiconductors, dry etching devices, wet etching devices, plasma etching devices, reactive ion etching devices, reactive ion beam etching devices, sputter etching devices, ion beam etching devices, diffusion and oxidation devices, sputtering devices, ashing devices, plasma ashing devices, cleaning devices, ion implantation devices, plasma CVD devices, exhaust devices, exposure devices, grinding devices, film-forming devices, dry-etching cleaning devices, UV/O3 cleaning devices, ion beam cleaning devices, laser beam cleaning devices, plasma cleaning devices, gas etching cleaning devices, extraction cleaning devices, Soxhlet extraction cleaning devices, high-temperature high-pressure extraction cleaning devices, microwave extraction cleaning devices, supercritical extraction cleaning devices, cleaning devices using hydrofluoric acid, hydrochloric acid, sulfuric acid, or ozonated water, steppers, coaters and developers, CMP devices, excimer laser exposure devices, chemical liquid pipes, gas pipes, devices involving plasma treatment such as NF3 plasma treatment, O2 plasma treatment, or fluorine plasma treatment, heating film-forming devices, wafer transporting devices, wafer cleaning devices, silicon wafer cleaning devices, silicon wafer processing devices, devices used in LP-CVD, devices used in lamp annealing, and devices used in reflow.

Specific examples of applications in the field relating to semiconductors include seal materials such as O-rings and gaskets for gate valves, quartz windows, chambers, chamber lids, gates, bell jars, couplings, and pumps; seal materials such as O-rings, hoses, and tubes for resist developers and strippers; lining and coating of resist developer tanks, stripper tanks, wafer cleaning liquid tanks, and wet etching tanks; diaphragms of pumps; rolls for transporting wafers; hoses and tubes for wafer cleaning liquids; seal materials for clean equipment, such as sealants for clean equipment such as cleanrooms; sealing materials for storage cabinets for devices such as semiconductor manufacturing devices and wafers; and diaphragms for transporting chemical liquids used in production of semiconductors.

In the field of automobiles, the molded article of the disclosure may be used for engine bodies, main drive systems, valve train systems, lubrication and cooling systems, fuel systems, intake and exhaust systems, transmission systems of driveline systems, steering systems and braking systems of chassis, and electrical components such as basic electrical parts, electrical parts of control systems, and electrical accessories. The field of automobiles also relates to motorcycles.

In relation to the aforementioned engine bodies and peripherals thereof, the molded article of the disclosure may be used for seal materials requiring heat resistance, oil resistance, fuel oil resistance, resistance to antifreeze for engine cooling, and steam resistance. Examples of such seal materials include seals such as gaskets, shaft seals, and valve stem seals, non-contact or contact packings such as self-seal packings, piston rings, split ring packings, mechanical seals, and oil seals, bellows, diaphragms, hoses, tubes, and seal materials used for electric wires, cushioning materials, damping materials, and belt AT devices.

Specific examples of applications in the fuel systems include O-rings used for fuel injection systems, cold start injectors, quick connectors of fuel lines, sender flange quick connectors, fuel pumps, fuel tank quick connectors, gasoline mixing pumps, gasoline pumps, tube bodies of fuel tubes, connectors of fuel tubes, and injectors; seals used for exhaust manifolds, fuel filters, pressure control valves, canisters, caps of fuel tanks, fuel pumps, fuel tanks, sender units of fuel tanks, fuel injection systems, high pressure fuel pumps, fuel line connector systems, pump timing control valves, suction control valves, solenoid sub-assemblies, and fuel cut valves; canister purge solenoid valve seals, onboard refueling vapor recovery (ORVR) valve seals, oil seals for fuel pumps, fuel sender seals, fuel tank roll over valve seals, filler seals, injector seals, filler cap seals, and seals of filler cap valves; hoses such as fuel hoses, fuel supply hoses, fuel return hoses, vapor (evaporator) hoses, vent (breather) hoses, filler hoses, filler neck hoses, hoses inside fuel tanks (in-tank hoses), control hoses of carburetors, fuel inlet hoses, and fuel breather hoses; gaskets used for fuel filters and fuel line connector systems, and flange gaskets used for carburetors; lining materials for vapor recovering lines, fuel feed lines, and vapor ORVR lines; diaphragms used for canisters, ORVR, fuel pumps, fuel tank pressure sensors, gasoline pumps, sensors of carburetors, combined air controlling (CAC) systems, pulsation dampers, canisters, and auto-valves, and pressure regulator diaphragms of fuel injection systems; valves for fuel pumps, carburetor needle valves, roll over check valves, and check valves; tubes used in vents (breathers) and fuel tanks; tank packings of, for example, fuel tanks, and packings of acceleration pump pistons of carburetors; fuel sender damping parts for fuel tanks; O-rings and diaphragms for regulating fuel pressure; accelerator pump cups; in-tank fuel pump mounts; injector cushion rings of fuel injection systems; injector seal rings; needle valve cores of carburetors; acceleration pump pistons of carburetors; valve sheets of combined air controlling (CAC) systems; fuel tank bodies; and sealing parts for solenoid valves.

Specific examples of applications in the brake systems include diaphragms used for mastervacs, hydraulic brake hoses, air brakes, and brake chambers of air brakes; hoses used as brake hoses, brake oil hoses, and vacuum brake hoses; seal materials such as oil seals, O-rings, packings, and brake piston seals; air valves and vacuum valves for mastervacs, and check valves for brake valves; piston cups (rubber cups) for master cylinders and brake cups; and O-rings and grommets for master cylinders and vacuum boosters of hydraulic brakes, boots for wheel cylinders of hydraulic brakes, and anti-lock brake systems (ABS).

Specific examples of applications in the basic electrical parts include insulators and sheaths of electric wires (harnesses), tubes of harness exterior parts, and grommets for connectors.

Specific examples of applications in the electrical parts of control systems include coating materials of various sensor lines.

Specific examples of applications in the electrical accessories include O-rings and packings of automobile air conditioners, cooler hoses, high-pressure air conditioner hoses, air conditioner hoses, gaskets for electronic throttle units, plug boots for direct ignition, and diaphragms for distributors. Further, the molded article of the disclosure may be used for bonding of electric parts.

Specific examples of applications in the intake and exhaust systems include packings used for intake manifolds and exhaust manifolds and throttle body packings of throttles; diaphragms used for exhaust gas recirculation (EGR) systems, pressure control (BPT) systems, wastegates, turbocharger wastegates, actuators, actuators of variable turbine geometry (VTG) turbochargers, and exhaust purifying valves; hoses such as control hoses of exhaust gas recirculation (EGR) systems, emission control hoses, turbo oil hoses (feed side), turbo oil hoses (return side), turbo air hoses, and intercooler hoses of turbochargers, turbocharger hoses, hoses connected with compressors of turbo engines equipped with intercoolers, exhaust gas hoses, air intake hoses, turbo hoses, and diesel particulate filter (DPF) sensor hoses; air ducts and turbo air ducts; intake manifold gaskets; and seal materials of EGR systems, valve sheets for preventing after burn of AB valves, turbine shaft seals (of turbochargers, for example), and seal parts used for groove parts of rocker covers and air intake manifolds used in engines of automobiles.

In addition, with respect to the exhaust gas control parts, the molded article may be used as any of seals used for vapor recovery canisters, catalytic converters, exhaust gas sensors, and oxygen sensors and seals for solenoid armatures of vapor recovery and vapor canisters; and intake manifold gaskets.

With respect to the diesel engine-related parts, the molded article may be used as any of O-ring seals for direct injectors, rotary pump seals, control diaphragms, fuel hoses, EGR systems, priming pumps, and diaphragms of boost compensators. The molded article may also be used for O-rings, seal materials, hoses, tubes, and diaphragms used in urea SCR systems, urea solution tank bodies of urea SCR systems, and seal materials of urea solution tanks.

Specific examples of applications in the transmission systems include transmission-related bearing seals, oil seals, O-rings, packings, and torque converter hoses.

Examples of applications also include mission oil seals, mission oil hoses, ATF hoses, O-rings, and packings of AT.

Examples of the transmission include automatic transmission (AT), manual transmission (MT), continuously variable transmission (CVT), and dual clutch transmission (DCT).

Examples of applications also include oil seals, gaskets, O-rings, and packings for manual or automatic transmissions, oil seals, gaskets, O-rings, and packings for (belt-type or toroidal-type) continuously variable transmissions, packings for ATF linear solenoids, oil hoses for manual transmissions, ATF hoses for automatic transmissions, and CVTF hoses for (belt-type or toroidal-type) continuously variable transmissions.

Specific examples of applications in the steering systems include power steering oil hoses and high-pressure power steering hoses.

Examples of applications used in engine bodies of automobile engines include gaskets such as cylinder head gaskets, cylinder head cover gaskets, oil pan packings, and general gaskets, seals such as O-rings, packings, and timing belt cover gaskets, hoses such as control hoses, damper rubbers of engine mounts, control valve diaphragms, and camshaft oil seals.

Examples of applications in the main drive systems of automobile engines include shaft seals such as crankshaft seals and camshaft seals.

Examples of applications in the valve train systems of automobile engines include valve stem oil seals of engine valves and valve sheets of butterfly valves.

Examples of applications in the lubrication and cooling systems of automobile engines include engine oil cooler hoses of engine oil coolers, oil return hoses, seal gaskets, water hoses used around radiators, seals of radiators, gaskets of radiators, O-rings of radiators, vacuum pump oil hoses of vacuum pumps, radiator hoses, radiator tanks, diaphragms for oil pressure, and fan coupling seals.

As mentioned above, specific examples of applications in the field of automobiles include engine head gaskets, oil pan gaskets, manifold packings, seals for oxygen sensors, oxygen sensor bushes, seals for nitrogen oxide (NOx) sensors, nitrogen oxide (NOx) sensor bushes, seals for sulfur oxide sensors, seals for temperature sensors, temperature sensor bushes, seals for diesel particulate filter sensors, diesel particulate filter sensor bushes, injector O-rings, injector packings, O-rings and diaphragms of fuel pumps, gearbox seals, power piston packings, seals of cylinder liners, seals of valve stems, static valve stem seals, dynamic valve stem seals, front pump seals of automatic transmissions, rear axle pinion seals, gaskets of universal joints, pinion seals of speedometers, piston cups of foot brakes, O-rings and oil seals of torque transmission systems, seals and bearing seals of exhaust gas re-combustion systems, hoses for re-combustion systems, diaphragms for sensors of carburetors, damper rubbers (e.g., engine mounts, exhaust parts, muffler hangers, suspension bushes, center bearings, strut bumper rubbers), damper rubbers (e.g., strut mounts, bushes) for suspensions, drive system damper rubbers (e.g., dampers), fuel hoses, tubes and hoses of EGR systems, twin carburetor tubes, cores of needle valves of carburetors, flange gaskets of carburetors, oil hoses, oil cooler hoses, ATF hoses, cylinder head gaskets, water pump seals, gearbox seals, needle valve tips, reeds of reed valves for motorcycles, oil seals of automobile engines, seals of gasoline hose guns, seals for automobile air conditioners, rubber hoses for intercoolers of engines, seals of fuel line connector systems, CAC valves, needle tips, electric wires around engines, filler hoses, automobile air conditioner O-rings, intake gaskets, fuel tank materials, diaphragms for distributors, water hoses, clutch hoses, PS hoses, AT hoses, mastervac hoses, heater hoses, air conditioner hoses, ventilation hoses, oil filler caps, PS rack seals, rack and pinion boots, CVJ boots, ball joint dust covers, strut dust covers, weather strips, glass run channels, center unit packings, body side welts, bumper rubbers, door latches, dash insulators, high tension cords, flat belts, poly V-belts, timing belts, toothed belts, V-ribbed belts, tires, wiper blades, diaphragms and plungers for regulators of LPG vehicles, diaphragms and valves for regulators of CNG vehicles, DME-resistant rubber parts, diaphragms and boots of automatic belt tensioners, diaphragms and valves for idle speed control, actuators for cruise control, diaphragms, check valves, and plungers of negative-pressure pumps, diaphragms and O-rings of O.P.S., gasoline pressure relief valves, O-rings and gaskets of engine cylinder sleeves, O-rings and gaskets of wet cylinder sleeves, seals and gaskets of differential gears (seals and gaskets for gear oils), seals and gaskets of power steering systems (seals and gaskets of PSF), seals and gaskets of shock absorbers (seals and gaskets of SAF), seals and gaskets of constant-velocity joints, seals and gaskets of wheel bearings, coatings for metal gaskets, caliper seals, boots, wheel bearing seals, and bladders used in vulcanization molding of tires.

In the fields of aircraft, space and rockets, and shipping, the molded article may especially be used in the fuel systems and the lubrication systems.

In the field of aircraft, the molded article may be used as, for example, any of seal parts for aircraft, parts for aircraft used in relation to engine oils for aircraft, jet engine valve stem seals, gaskets, O-rings, rotary shaft seals, gaskets of hydraulic equipment, fire wall seals, hoses, gaskets, and O-rings for fuel feed, and cables, oil seals, and shaft seals for aircraft.

In the field of space and rockets, the molded article may be used as, for example, any of lip seals, diaphragms, and O-rings of spacecraft, jet engines, and missiles, O-rings resistant to oils for gas turbine engines, and damper stage pads for ground level control of missiles.

In the field of shipping, the molded article may be used as, for example, any of propeller shaft stern seals of screws, valve stem seals for intake and exhaustion of diesel engines, valve seals of butterfly valves, valve sheets and shaft seals of butterfly valves, shaft seals of butterfly valves, stern tube seals, fuel hoses and gaskets, O-rings for engines, cables for shipping, oil seals for shipping, and shaft seals for shipping.

In the field of chemistry such as chemical plants and the field of chemicals such as pharmaceuticals, the molded article may be used in steps requiring high-level chemical resistance, such as steps of manufacturing chemicals, including pharmaceuticals, agrochemicals, coating materials, and resins.

Specific examples of applications in the fields of chemistry and chemicals include: seals used for chemical devices, pumps for chemicals, flow meters, pipes for chemicals, heat exchangers, agrochemical sprayers, agrochemical transporting pumps, gas pipes, fuel cells, analyzers and physical and chemical instruments (e.g., column fittings of analyzers and meters), expansion joints of flue-gas desulfurization devices, nitric acid plants, and turbines of power plants, seals used in medical sterilization processes, seals for plating solutions, runner seals of belts for papermaking, and joint seals of wind tunnels; O-rings used in chemical devices such as reactors and stirrers, analyzers and meters, chemical pumps, pump housings, valves, and tachometers, O-rings for mechanical seals, and O-rings for compressor sealing; packings used in high-temperature vacuum dryers and tube joints of gas chromatographs and pH meters, and glass cooler packings of sulfuric acid manufacturing devices; diaphragms used in diaphragm pumps, analyzers, and physical and chemical instruments; gaskets used in analyzers and meters; ferrules used in analyzers and meters; valve sheets; U-cups; linings used in chemical devices, gasoline tanks, and wind tunnels, and corrosion-resistant linings of tanks for anodizing on aluminum; coatings of masking jigs for plating; valve parts of analyzers and physical and chemical instruments; expansion joints of flue-gas desulfurization plants; hoses resistant to acids such as concentrated sulfuric acid, chlorine gas transporting hoses, oil-resistant hoses, and rainwater drainage hoses of benzene or toluene storage tanks; chemical-resistant tubes used in analyzers and physical and chemical instruments and medical tubes; trichloroethylene-resistant rolls for fiber dyeing and rolls for dyeing; stoppers for pharmaceuticals; medical rubber stoppers; chemical bottles, chemical tanks, bags, and chemical containers; strong acid-resistant and solvent-resistant protective items such as gloves and boots.

In the field of photography such as film processors, the field of printing such as printers, and the field of coatings such as coating equipment, the molded article may be used as any of rolls, belts, seals, and valve parts of dry copiers.

Specific examples of applications in the field of photography, the field of printing, and the field of coatings include surface layers of transfer rollers of copiers, cleaning blades of copiers, and belts of copiers; rolls (e.g., fixing rolls, crimp rolls, and pressure rolls) and belts for OA equipment such as copiers, printers, and fax machines; rolls, roll blades, and belts of PPCs; rolls of film processors and X-ray film processors; printing rolls, scrapers, tubes, valve parts, and belts of printing equipment; ink tubes, rolls, and belts of printers; application rolls, scrapers, tubes, and valve parts of application or coating equipment; processing rolls, gravure rolls, guide rolls, guide rolls of coating lines for manufacturing of magnetic tapes, gravure rolls of coating lines for manufacturing of magnetic tapes, and coating rolls.

In the field of food machinery such as food plant equipment and household items, the molded article may be used in steps of food production, food transportation, and food storage.

Specific examples of applications in the field of food machinery include seals of plate-type heat exchangers, solenoid valve seals of vending machines, packings of thermo pots, sanitary pipe packings, packings of pressure cookers, seals of boilers, gaskets for heat exchangers, diaphragms and packings for food processing equipment, rubber materials (e.g., seals such as heat exchanger gaskets, diaphragms, and O-rings, pipes, hoses, sanitary packings, valve packings, packings for filling used as joints between the mouth of a container such as a bottle and a filler) for food processing equipment. The molded article may also be used as packings, gaskets, tubes, diaphragms, hoses, and joint sleeves used for products such as alcohols and soft drinks, filling devices, food sterilizers, brewing devices, boilers, and food vending machines.

In the field of equipment for nuclear power plants, the molded article may be used as, for example, any of check valves and reducing valves around reactors and seals of devices for concentration of uranium hexafluoride.

Specific examples of applications in the field of general industry include seal materials for hydraulic devices such as machine tools, construction machinery, and hydraulic machines; seals and bearing seals of hydraulic, lubricating machinery; seal materials used in mandrels; seals used for windows of dry cleaning devices; seals and (vacuum) valve seals of cyclotrons, seals of proton accelerators, seals of automatic wrapping machines, diaphragms of pumps for analyzers (air pollution monitoring devices) for sulfurous acid gas or chlorine gas in the air, snake pump lining, rolls and belts of printers, belts (conveyor belts) for transportation, squeeze rolls for pickling of sheet steel, cables of robots, solvent squeezing rolls in aluminum rolling lines, O-rings of couplers, acid-resistant cushioning materials, dust seals and lip rubbers of sliding portions of cutting machinery, gaskets of garbage incinerators, friction materials, metal or rubber surface modifiers, and covering materials. The molded article may also be used as gaskets and seal materials of devices used in papermaking processes, sealing agents of filter units for cleanrooms, sealing agents for construction, protective coatings for concrete and cement, glass cloth impregnating materials, processing aids for polyolefins, moldability improving additives for polyethylene, fuel containers of small generators and lawn mowers, and pre-coated metals prepared by primer-treating metal plates. Also, fabrics impregnated and sintered may be used as sheets or belts.

Specific examples of applications in the field of steel include sheet steel processing rolls of sheet steel processing equipment.

Specific examples of applications in the field of electrics include insulating oil caps of Shinkansen, venting seals of liquid-immersed transformers, seals of transformers, jackets of oil well cables, seals of ovens such as electric furnaces, window frame seals of microwave ovens, seal materials used in bonding wedges and necks of CRTs, seal materials of halogen lamps, fixing agents for electric parts, seal materials for treating terminals of sheathed heaters, and seal materials used in insulating and damp-proofing treatment on wire terminals of electrical devices. The molded article may also be used as covering materials for oil- and heat-resistant electric wires, highly heat-resistant electric wires, chemical-resistant electric wires, highly insulating electric wires, high voltage power lines, cables, electric wires used in geothermal power generation devices, and electric wires used around automobile engines. The molded article may also be used as any of oil seals and shaft seals of cables for vehicles. The molded article may also be used as any of electrically insulating materials (e.g., materials used for insulating spacers of electric devices, insulating tapes used at joints and ends of cables, and heat-shrinkable tubes) and materials for electric and electronic devices used in high-temperature atmosphere (e.g., lead wire materials for motors and electric wire materials used around high-temperature furnaces). The molded article may also be used as any of sealing layers and protecting films (back sheets) of solar cells.

In the field of fuel cells, the molded article may be used as, for example, any of seal materials between electrodes or between an electrode and a separator in polymer electrolyte fuel cells and phosphoric acid salt fuel cells, and seals, packings, and separators of pipes for hydrogen, oxygen, or generated water.

In the field of electronic parts, the molded article may be used as, for example, any of heat-radiating materials, electromagnetic-wave-shielding materials, and gaskets for hard disk drives (magnetic recording devices) of computers. The molded article may also be used as shock-absorbing rubbers (crash stoppers) of hard disk drives, binders for electrode active materials of nickel hydrogen secondary batteries, binders for active materials of lithium ion batteries, polymer electrolytes of lithium secondary batteries, binders for positive electrodes of alkaline storage batteries, binders for EL elements (electroluminescent elements), binders, seal materials, and sealing agents for electrode active materials of capacitors, covering materials for quartz of optical fibers, films and sheets such as covering materials for optical fibers, potting, coating, or bonding seals for electronic parts and circuit boards, fixing agents for electronic parts, modifiers for seal materials such as epoxy compounds, coatings for printed circuit boards, modifiers for printed circuit board prepreg resins such as epoxy compounds, scattering inhibitors for electric light bulbs, gaskets for computers, large computer cooling hoses, packings such as gaskets and O-rings for secondary batteries, especially lithium secondary batteries, sealing layers, connectors, and dampers covering one or both of outside surfaces of organic EL structures.

In the field of equipment for transporting chemicals, the molded article may be used as, for example, any of safety valves and loading valves of trucks, trailers, tank trucks, and shipping.

In the field of parts of equipment for prospecting and mining energy sources such as petroleum and gas, the molded article may be used as, for example, any of seal materials used in mining petroleum or natural gas and boots of electric connectors used in oil wells.

Specific examples of applications in the field of parts of equipment for prospecting and mining energy sources include drill bit seals, pressure-control diaphragms, seals of horizontal drilling motors (stators), stator bearing (shaft) seals, seal materials used in blowout preventers (BOP), seal materials used in rotary blowout preventers (pipe wipers), seal materials and gas-liquid connectors used in measurement while drilling systems (MWD), logging tool seals (e.g., O-rings, seals, packings, liquid-gas connectors, and boots) used in logging equipment, expandable packers and completion packers, and packer seals used therefor, seals and packings used in cementing devices, seals used in perforators (perforating devices), seals, packings, and motor linings used in mud pumps, covers of underground sound inspection devices, U-cups, composition seating cups, rotary seals, laminate elastomeric bearings, seals for flow control, seals for sand control, seals of safety valves, seals of hydraulic fracturing equipment, seals and packings of linear packers and linear hangers, seals and packings of well heads, seals and packings of chokes and valves, seal materials for logging while drilling (LWD) systems, diaphragms (e.g., diaphragms for feeding lubricants in petroleum mining pits) used in prospecting and mining petroleum, gate valves, electronic boots, and seal elements of perforating guns.

The molded article may also be used as, for example, any of joint seals in kitchens, bathrooms, and lavatories; fabrics of outdoor tents; seal materials for materials of stamps; rubber hoses for gas heat pumps and Freon-resistant rubber hoses; films, linings, and weather-resistant covers for agriculture; and tanks of laminated sheet steel used in the field of construction or home appliances.

The molded article may also be used as an article bonded to a metal such as aluminum. Examples of such applications include door seals, gate valves, pendulum valves, and solenoid tips, as well as piston seals and diaphragms bonded to metal and metal rubber parts such as metal gaskets bonded to metal.

The molded article of the disclosure may also be used as any of rubber parts, brake shoes, and brake pads of bicycles.

Another exemplary form of the molded article is a belt. Examples of the belt include the following: power transmission belts (including flat belts, V-belts, V-ribbed belts, toothed belts), and transportation belts (conveyor belts) such as flat belts used for portions exposed to high temperatures, such as portions around engines of agricultural machinery, machine tools, and industrial machinery; conveyor belts for transporting scattered matters or particles of coal, smashed rock, earth and sand, ores, and wood chips at high temperatures; conveyor belts used in iron mills, such as blast furnaces; conveyor belts used for applications exposed to high temperatures in high precision machine assembling factories and food factories; V-belts and V-ribbed belts for agricultural machinery, general equipment (e.g., OA equipment, printers, dryers for business purposes), and automobiles; power transmission belts of transporting robots; toothed belts such as power transmission belts of food machinery and machine tools; and toothed belts for automobiles, OA equipment, medical uses, and printers.

In particular, timing belts are typical toothed belts for automobiles.

The above belt may have a single layer structure or a multi-layer structure.

In the case of a multi-layer structure, the belt may have a layer obtainable by cross-linking the composition of the disclosure and a layer of another material.

Examples of the layer of another material in the multi-layer belt include layers formed from different rubber, layers formed from thermoplastic resin, fiber-reinforced layers, canvas layers, and metal foil layers.

The molded article of the disclosure may also be used as damper pads for industrial use, damper mats, slab mats for railways, pads, and damper rubbers for automobiles. The damper rubbers for automobiles may be damper rubbers for engine mounts, motor mounts, member mounts, strut mounts, bushes, dampers, muffler hangers, and center bearings.

Examples of other applications include joint parts such as flexible joints and expansion joints, boots, and grommets. In the field of shipping, the molded article may be used for marine pumps.

The joint parts are joints used for pipes and piping equipment, and are used for preventing vibrations and noises generated by piping systems, absorption of expansion and contraction or displacement due to temperature change and pressure change, absorption of dimensional changes, and mitigation or prevention of influences due to earthquakes or land subsidence.

The flexible joints and expansion joints may be preferably used as molded articles with complicated shapes for shipbuilding piping, piping of machinery such as pumps and compressors, chemical plant piping, electric piping, piping of civil engineering works and waterworks, and automobiles.

The boots may be preferably used as molded articles with complicated shapes, such as boots for various industries, including boots for automobiles such as constant-velocity joint boots, dust covers, rack and pinion steering boots, pin boots, and piston boots, boots for agricultural machinery, boots for industrial vehicles, boots for construction machinery, boots for hydraulic machinery, boots for pneumatic machinery, boots for centralized lubrication systems, boots for liquid transportation, boots for firefighting, and boots for liquefied gas transportation.

The molded article of the disclosure may also be used as any of diaphragms for filter presses, diaphragms for blowers, diaphragms for water supply, diaphragms for liquid storage tanks, diaphragms for pressure switches, diaphragms for accumulators, and diaphragms for air springs such as suspensions.

The molded article of the disclosure is added to rubber or resin, thereby providing an anti-slip agent capable of providing a molded article or coating film that is less slippery in environments wet with water such as rain, snow, ice, and sweat.

The molded article of the disclosure may also be used as a cushioning material for heat-press molding in production of decorative plywood, printed circuit boards, electrically insulated plates, and hard polyvinyl chloride laminates from melamine resin, phenol resin, or epoxy resin.

The molded article of the disclosure may also contribute to give impermeability to various supporters such as sealing gaskets related to weapons and protective clothing against contact with invasive chemicals.

The molded article of the disclosure may also be used as any of O-rings (square-rings), V-rings, X-rings, packings, gaskets, diaphragms, oil seals, bearing seals, lip seals, plunger seals, door seals, lip and face seals, gas delivery plate seals, wafer support seals, barrel seals, and other seal materials for sealing lubricants (e.g., engine oil, mission oil, gear oil), fuel oils, or greases (in particular, urea grease) containing amine additives (in particular, amine additives used as antioxidants, detergents, or dispersants) used in transports such as automobiles and shipping. The molded article may also be used as any of tubes, hoses, rubber rolls, coatings, belts, and valve bodies of valves. The molded article may also be used as any of laminating materials and lining materials.

The molded article may also be used as any of covering materials for heat- and oil-resistant electric wires used as, for example, electric wires of sensors contacting transmission oil and/or engine oil in internal combustion engines of automobiles and detecting the oil temperature and/or the oil pressure, and may be used in high-temperature environment such as the inside of oil pans of automatic transmissions or engines.

Moreover, a vulcanized coating may be formed on the molded article of the disclosure before use. Specific examples of such applications include non-viscous oil-resistant rolls for copiers, weather-resistant freeze-preventive weather strips, rubber stoppers for infusion solution, vial rubber stoppers, release agents, non-viscous light-duty transport belts, adhesion-preventive coatings of pulley gaskets of automobile engine mounts, covering processing of synthetic fibers, and bolt parts or joints having a packing covering thin layer.

The applications of the molded article of the disclosure with respect to the automobile-related parts include motorcycle parts having the same structures.

Examples of the automobile-related fuels include light oil, gasoline, and fuels for diesel engines (including biodiesel fuel).

The composition of the disclosure can also be used as various parts in various industrial fields, in addition to the use in the form of a molded article obtainable by cross-linking the composition. Next, applications of the composition of the disclosure will be described.

The composition of the disclosure can be used as any of surface modifiers for metal, rubber, plastic, or glass; sealants and covers requiring heat resistance, chemical resistance, oil resistance, and non-viscousness, such as metal gaskets and oil seals; and non-viscous covers or bleed barriers for rolls for OA equipment and belts for OA equipment, and may be used for impregnation or bake-application of/to fabric sheets and belts.

The composition of the disclosure, with a high viscosity and high concentration, may be formed into any of seal materials, linings, and sealants having more complicated shapes by a usual method. The composition of the disclosure, with a low viscosity, may be formed into thin films of several micrometers. The composition of the disclosure, with a middle viscosity, may be applied to any of pre-coated metals, O-rings, diaphragms, and reed valves.

Further, the composition of the disclosure may be applied to any of rolls or belts for transporting fabric or paper, printing belts, chemical-resistant tubes, stoppers for chemicals, and fuel hoses.

Examples of the material substrates to be covered with the composition of the disclosure include metals such as iron, stainless steel, copper, aluminum, and brass; glass products such as glass plates and woven or nonwoven fabric of glass fiber; molded articles of or articles covered with general-purpose or heat-resistant resin (e.g., polypropylene, polyoxymethylene, polyimide, polyamide imide, polysulfone, polyether sulfone, and polyether ether ketone); molded articles of or articles covered with general-purpose rubber (e.g., SBR, butyl rubber, NBR, EPDM) or heat-resistant rubber (e.g., silicone rubber, fluororubber); and woven or nonwoven fabric of natural or synthetic fiber.

Coated articles formed from the composition of the disclosure can be used in the fields requiring heat resistance, solvent resistance, lubricity, and non-viscousness. Examples of specific applications include rolls (e.g., fixing rolls, crimp rolls) and conveyor belts for OA equipment such as copiers, printers, and fax machines; sheets and belts; and O-rings, diaphragms, chemical-resistant tubes, fuel hoses, valve seals, gaskets for chemical plants, and engine gaskets.

The composition of the disclosure may be dissolved in a solvent and then used as a coating or an adhesive. The composition of the disclosure may also be used as a coating in the form of emulsion dispersion (latex).

The composition may be used as any of surface-treating agents for seal materials and linings for various devices and pipes, and structures formed of an inorganic or organic substrate such as metal, ceramic, glass, stone, concrete, plastic, rubber, wood, paper, or fiber.

The composition may be applied to any of the above substrates using a dispenser or by screen printing.

The composition of the disclosure may be used as a coating composition for casting a film or for immersing a substrate such as fabric, plastic, metal, or elastomer.

In particular, the composition of the disclosure in the form of latex may be used for production of covered fabric, protective gloves, impregnated fibers, covers for O-rings, covers for quick connecting O-rings for fuel systems, covers for fuel system seals, covers for fuel tank rollover valve diaphragms, covers for fuel tank pressure sensor diaphragms, covers for oil filter and fuel filter seals, covers for fuel tank sender seals and for sender head fitting seals, covers for rolls of fixing mechanisms of copiers, and polymer coating compositions.

These compositions are useful for covering silicone rubber, nitrile rubber, and other elastomers. In order to improve the heat stability thereof, as well as the permeation resistance and chemical resistance of substrate elastomers, these compositions are useful for covering parts produced from such elastomers. Examples of other applications include coverings for heat exchangers, expansion joints, vats, tanks, fans, flue ducts, and other ducts, and housing structures (e.g., concrete housing structures). The composition may be applied to exposed cross sections of multi-layer parts in, for example, a production method for hose-like structures or diaphragms. Sealing materials at jointing portions and coupling portions are often formed of a hard material, and the composition of the disclosure provides improved frictional interfaces and improved dimension interference fit with a reduced, slight amount of leakage along the sealed surface. The latex thereof improves the seal durability in applications of various automobile systems.

These materials can be used in production of any of power steering systems, fuel systems, air conditioning systems, and bonding portions where hoses and tubes are coupled with other parts. The composition shows its usefulness in repair of production defects (and damages due to use) in multi-layer rubber structures such as three-layer fuel hoses. The composition is also useful for application to thin sheet steel which may be formed or embossed before or after the application of the coating. For example, multiple layers of covered steels may be assembled and a gasket may be disposed between two rigid metal parts. The sealing effect can be achieved by applying the composition of the disclosure between the layers. This process may be used for producing engine head gaskets and exhaust manifold gaskets so as to decrease the bolt force and strain of assembled parts, while providing good saving and low release of fuel owing to little cracking, bending, and hole deformation.

The composition of the disclosure may also be used as coatings; substrate-integrated gaskets and packings formed by dispenser-molding the composition on substrates including inorganic materials such as metal or ceramic; and multi-layer articles prepared by covering substrates including inorganic materials such as metal or ceramic.

### EXAMPLES

Next, the disclosure will be described in more detail with reference to examples, but the disclosure is not limited only to these examples.

### Production Example 1

An aqueous dispersion of a TFE/PMVE/CF₂=CFOCF₂CF(CF₃)OCF₂CF₂CN (CNVE) copolymer was obtained according to Production Example 1 of WO 2020/026909 A.

The resulting aqueous dispersion in an amount of 3110 g was diluted with 3730 g of water, and slowly added to 3450 g of a 4.8% by mass nitric acid aqueous solution with stirring. After stirring for 30 minutes, the coagulate was separated by filtration, and the polymer obtained was thoroughly washed with water and dried in vacuum, whereby 680 g of a fluorine-containing elastomer was obtained.

According to ¹⁹F-NMR analysis, the fluorine-containing elastomer obtained had a monomer unit composition of TFE/PMVE/CNVE = 59.3/39.9/0.8 (mol%). According to the measurement by infrared spectroscopy, the characteristic absorption of the carboxyl group was observed near 1774.9 cm⁻¹ and 1808.6 cm⁻¹, and the characteristic absorption of the OH group was observed near 3557.5 cm⁻¹ and 3095.2 cm⁻¹.

The metal content of the fluorine-containing elastomer obtained was measured by the measuring method disclosed in WO 94/28394 A. Specifically, a predetermined amount of a sample containing a metal to be quantified was ashed in a cuvette under ashing conditions including an ashing temperature of about 1000°C and an ashing time of about 240 seconds, and then subjected to the measurement of absorbance using a flameless atomic absorption spectrophotometer. The metal content of the fluorine-containing elastomer obtained was 10 ppm or less.

The compound shown below was synthesized by the method disclosed in Journal of Polymer Science, Polymer Chemistry, Vol. 20, pp. 2381-2393 (1982).

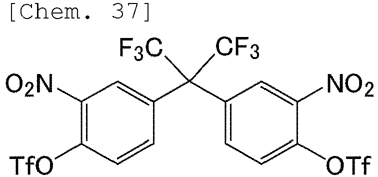

The compound (hereinafter referred to as Tf form) was used to synthesize the following cross-linking agents. The OTf group is a trifluoromethane sulfonate group (CF₃SO₃ group).

### Synthesis Example 1

To a 500-mL separable flask were added 300 g of toluene, 30 g of the Tf form, 23.9 g of 3,5-bis(trifluoromethyl)aniline, 39.6 g of cesium carbonate, 3.9 g of palladium acetate, and 14.6 g of triphenylphosphine in an argon stream, followed by reflux for 16 hours. After cooling, insoluble matter was filtered and toluene was distilled off under reduced pressure. The resulting substance was purified by column chromatography, whereby a compound with an LC purity of 99% represented by the following formula: 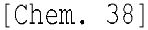 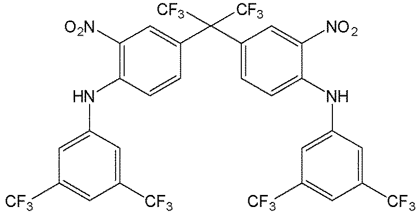 in an amount of 15.0 g (yield 40.6%) was obtained.

Next, a 1-L autoclave was charged with 15.0 g of the compound obtained previously, 1.5 g (dry equivalent) of 10%-Pd/C, and 260 g of ethanol, followed by nitrogen purge and hydrogen purge. The contents were stirred until the hydrogen kept at 0.5 MPa was no longer consumed, and stirring was further performed at 40°C for six hours. The catalyst was removed by filtration, and ethanol was distilled off under reduced pressure. The resulting substance was purified by column chromatography, whereby a target product with an LC purity of 99% represented by the following formula: 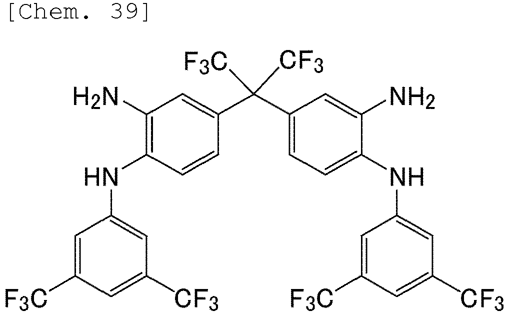 in an amount of 9.75 g (yield 65.0%) was obtained. The melting point of the target product measured by TG-DTA was 179°C.

### Synthesis Example 2

To a 1-L separable flask were added 60 g of the Tf form, 123 g of 2-aminopyridine, 79 g of cesium carbonate, and 720 g of toluene, followed by stirring at room temperature for two hours in an argon stream. To the mixture were further added 13.7 g of triphenylphosphine and 3.9 g of palladium acetate, and the temperature was raised to 70°C. After reaction at 70°C for three hours, the mixture was cooled to room temperature. The mixture was filtered and washed with isopropyl alcohol (IPA), whereby yellow crude crystals were obtained. To the crystals was added 180 g of methanol, followed by heating under reflux for 30 minutes. The resulting product was then cooled to room temperature, filtered, and washed with ion-exchanged water. After drying overnight at 70°C, a compound with an LC purity of 99% represented by the following formula: 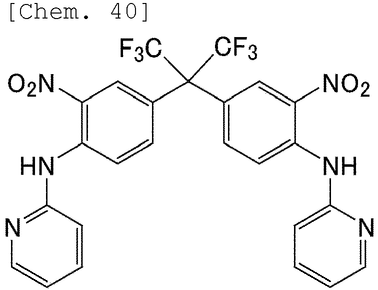 in an amount of 15.1 g (yield 30.0%) was obtained.

Next, a 1-L autoclave was charged with 40.0 g of the previously obtained compound, 2.0 g (dry equivalent) of 10%-Pd/C, and 240 g of THF, followed by nitrogen purge and hydrogen purge. The contents were stirred until the hydrogen kept at 0.5 MPa was no longer consumed, and stirring was further performed at 40°C for six hours. The catalyst was removed by filtration, and THF was distilled off under reduced pressure. To the resulting product was added ion-exchanged water for crystallization, followed by filtration and washing with ion-exchanged water, whereby light-pink crystals were obtained. The crystals were further dried under reduced pressure at 50°C, whereby a target product with an LC purity of 97.6% represented by the following formula: 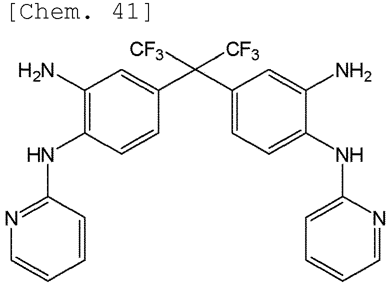 in an amount of 34.3 g (yield 95.0%) was obtained. The melting point of the target product measured by TG-DTA was 134°C.

### Synthesis Example 3

To a 200-ml separable flask were added 30.0 g of the Tf form and 101 g of 2,6-difluoroaniline, and the temperature was raised to 145°C. The mixture was maintained at 145°C for 48 hours, and then 105 g of isopropyl alcohol was added thereto at 70°C. The mixture was cooled to room temperature, and then filtered. After addition of isopropyl alcohol and subsequent stirring at 70°C for 30 minutes, the mixture was cooled to room temperature and then filtered. The resulting product was dried under reduced pressure at 70°C overnight, whereby a compound with an LC purity of 99.0% represented by the following formula: 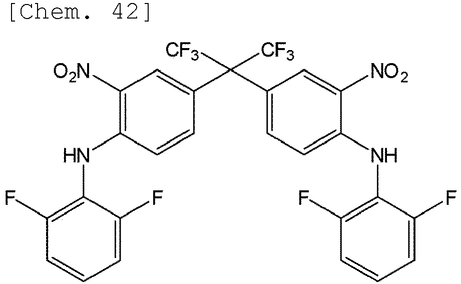 in an amount of 26.9 g (yield 95.3%) was obtained.

Next, a 1-L autoclave was charged with 63.5 g of the previously obtained compound, 3.2 g (dry equivalent) of 10%-Pd/C, and 318 g of THF, followed by nitrogen purge and hydrogen purge. The contents were stirred until the hydrogen kept at 0.5 MPa was no longer consumed, and stirring was further performed at 30°C for six hours. After removal of the catalyst by filtration, THF was distilled off under reduced pressure and then methanol was added, followed by filtration. The resulting product was washed with methanol, whereby light-pink crystals were obtained. The crystals were dried under reduced pressure at 50°C, whereby a target product with an LC purity of 98.4% represented by the following formula: 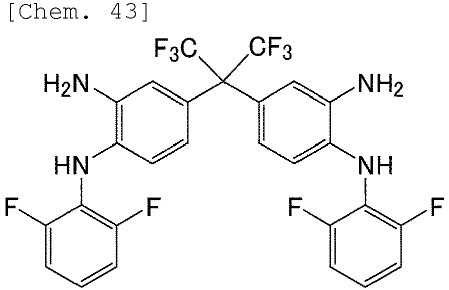 in an amount of 54.7 g (yield 95.0%) was obtained. The melting point of the target product measured by TG-DTA was 249°C.

### Synthesis Example 4

To a 500-ml separable flask were added 80.0 g of the Tf form, 249 g of o-toluidine, and 240 g of butyl acetate, and the contents were maintained at that temperature under reflux for one hour. After cooling, 720.0 g of IPA was added for crystallization, followed by filtration and subsequent washing with IPA, whereby orange crystals were obtained. The crystals were heated and refluxed using 240 g of isopropyl acetate for 30 minutes, followed by cooling to room temperature and subsequent filtration, whereby yellow crystals were obtained. The yellow crystals were dried at 50°C under reduced pressure overnight, whereby a compound with an LC purity of 98.1% represented by the following formula: 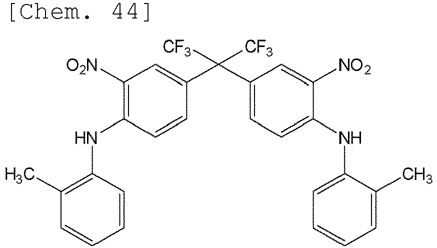 in an amount of 53.0 g (yield 75.7%) was obtained.

Next, a 1-L autoclave was charged with 60.0 g of the previously obtained compound, 4.5 g (dry equivalent) of 10%-Pd/C, and 300 g of THF, followed by nitrogen purge and hydrogen purge. The contents were stirred until the hydrogen kept at 0.5 MPa was no longer consumed, and stirring was further performed at 60°C for six hours. After removal of the catalyst by filtration, THF was distilled off under reduced pressure and then hexane was added for crystallization. The resulting product was filtered and washed with hexane, whereby light-yellow crystals were obtained. The crystals were dried under reduced pressure at 50°C, whereby a target product with an LC purity of 98.5% represented by the following formula: 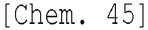 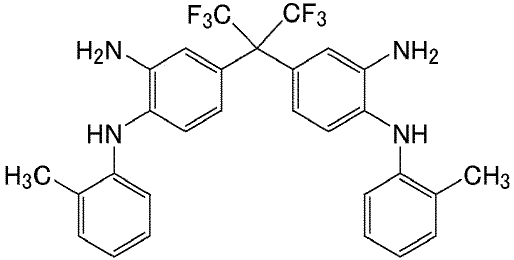 in an amount of 51.3 g (yield 95.0%) was obtained. The melting point of the target product measured by TG-DTA was 163°C.

### Example 1

The fluorine-containing elastomer obtained in Production Example 1, MT carbon black (available from Cancarb Limited, average particle size 0.3 µm) as a filler, and the compound (cross-linking agent) synthesized in Synthesis Example 2 were mixed at a mass ratio of 100/23/0.8 and kneaded in an open roll mill, whereby a cross-linkable fluororubber composition was prepared. This fluororubber composition was pressed at 200°C for 30 minutes and then heated in an oven at 290°C for 18 hours, whereby an O-ring (P24) test sample was produced. The cross-linkability of the fluororubber composition and the compression set of the test sample were measured by the following methods. Table 1 shows the results.

### (Cross-linkability)

The vulcanization properties of the fluororubber composition were analyzed using a rubber process analyzer 2000 available from Alpha Technologies under the conditions of 200°C for 30 minutes at a frequency of 1 Hz and a strain of 10%, and the G' maximum value and the appropriate vulcanization time (T₉₀) were determined.

### (Compression set, number of breakages)

The compression set of the test sample (O-ring (P24)) was measured in conformity with JIS K6262 at 290°C after 70 hours, 168 hours, 336 hours, and 504 hours. When a crack with a length of 10 mm or longer was found in the O-ring, it was judged as a breakage. The number of test samples with breakages was counted.

### Example 2

Samples were prepared as in Example 1 except that the cross-linking agent was changed to the compound synthesized in Synthesis Example 3. The cross-linkability and the compression set were measured. Table 1 shows the results.

### Example 3

Samples were prepared as in Example 1 except that the cross-linking agent was changed to the compound synthesized in Synthesis Example 4. The cross-linkability and the compression set were measured. Table 1 shows the results.

### Reference Example 1

Samples were prepared as in Example 1, except that the cross-linking agent was changed to 2,2-bis[3-amino-4-(N-phenylamino)phenyl]hexafluoropropane (melting point 183°C). The cross-linkability and the compression set were measured. Table 1 shows the results.

### (Heat resistance evaluation)

The test samples prepared in Examples 1 to 3 and Reference Example 1 were heated at 350°C for 21 hours.

The samples before heating and after heating were each immersed in a fluorine-based solvent R-318 (available from Daikin Industries, Ltd.) at 23°C for 96 hours. The swelling ratio after heating/before heating was determined. A smaller value means higher heat resistance. Table 1 shows the results.

**[Table 1]**

| | | | Example 1 | Example 2 | Example 3 | Reference Example 1 |
|---|---|---|---|---|---|---|
| Cross-linkability | | | | | | |
| | G max | kPa | 1259 | 1182 | 1234 | 1256 |
| | T90 | min | 16.21 | 14.39 | 6.72 | 8.21 |

| Compression set | | | | | | |
|---|---|---|---|---|---|---|
| | 70 h | % | 18 | 20 | 42 | 17 |
| | Number of breakages | | 0 | 0 | 0 | 0 |
| | 168 h | % | 25 | 27 | 57 | 25 |
| | Number of breakages | | 0 | 1 | 0 | 1 |
| | 336 h | % | 33 | 35 | 70 | 34 |
| | Number of breakages | | 0 | 0 | 0 | 0 |
| | 504 h | % | 38 | 41 | 77 | - |
| | Number of breakages | | 0 | 0 | 0 | 2 |

| Heat resistance evaluation | | | | | | |
|---|---|---|---|---|---|---|
| | Swelling ratio | % | 141 | 125 | 285 | 150 |

The results obtained show that the compound of the disclosure provides a cross-linked product with good crack resistance, while maintaining cross-linkability and heat resistance.

### Examples 4 to 6

The fluorine-containing elastomer obtained in Production Example 1, one of the compounds synthesized in Synthesis Examples 2 to 4 as a cross-linking agent, and Si₃N₄ as a cross-linking accelerator were mixed at a mass ratio of 100/0.8/0.1, followed by kneading in an open roll mill, whereby a cross-linkable fluororubber composition was prepared. The fluororubber composition was pressed at 180°C for 30 minutes, and then heated in an oven at 290°C for 18 hours, whereby an O-ring (P24) test sample was produced.

The plasma resistance of the test sample was measured by the following method. Table 2 shows the results.

### (Plasma resistance)

The test sample was subjected to irradiation with oxygen plasma or CF₄ plasma under the following conditions, and the weight loss per unit time was examined.

### Oxygen plasma irradiation conditions:

Gas flow rate: 16 sccm,
RF output: 400 W,
Pressure: 2.6 Pa,
Exposure time: 90 minutes, sampling every 15 minutes.

### CF₄ plasma irradiation conditions:

Gas flow rate: 16 sccm,
RF output: 400 W,
Pressure: 2.66 Pa,
Exposure time: 90 minutes, sampling every 15 minutes.

### Reference Example 2

A test sample was prepared as in Examples 4 to 6, except that the cross-linking agent was changed to 2,2-bis[3-amino-4-(N-phenylamino)phenyl]hexafluoropropane. The plasma resistance of the test sample was evaluated. Table 2 shows the results.

**[Table 2]**

| | | | Example 4 | Example 5 | Example 6 | Reference Example 2 |
|---|---|---|---|---|---|---|
| Oxygen plasma | | | | | | |
| | Weight reduction rate | wt%/min | 0.093 | 0.094 | 0.097 | 0.103 |

| CF₄ plasma | | | | | | |
|---|---|---|---|---|---|---|
| | Weight reduction rate | wt%/min | 0.034 | 0.033 | 0.032 | 0.035 |

The results obtained show that the composition of the disclosure provides a cross-linked product with good plasma resistance, in particular excellent resistance to oxygen plasma.

### INDUSTRIAL APPLICABILITY

The compound of the disclosure can be used as a cross-linking agent that provides a cross-linked product with excellent plasma resistance while maintaining heat resistance, or as a monomer for super engineering plastics.

## Claims

1. A compound represented by the following formula (1): 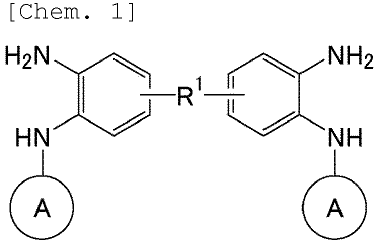 wherein
R¹ is -SO₂-, -O-, -CO-, an optionally substituted alkylene group, a group represented by the following formula: 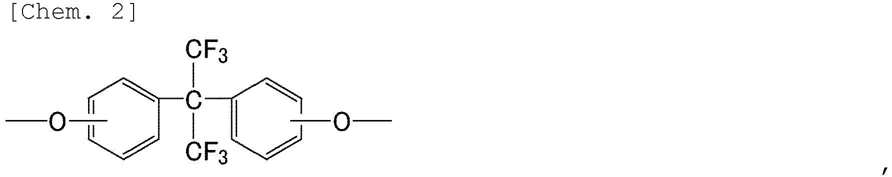 or a single bond; and
two As are each independently an aromatic ring group containing a 5- or 6-membered aromatic ring and optionally containing a substituent, where the aromatic ring group contains a substituent in the case where the aromatic ring group is a monocyclic aromatic ring group containing a 6-membered aromatic hydrocarbon ring.

2. The compound according to claim 1,
wherein the aromatic ring group is a monocyclic aromatic ring group containing a 6-membered aromatic hydrocarbon ring and containing a substituent or a monocyclic aromatic ring group containing a 5- or 6-membered aromatic heterocycle containing a nitrogen atom or a sulfur atom.

3. The compound according to claim 1 or 2,
wherein the aromatic ring group is a group having a greater n-electron deficiency than a benzene ring.

4. The compound according to any one of claims 1 to 3,
wherein the substituent includes at least one selected from the group consisting of a halogen atom and an alkyl group optionally containing a halogen atom.

5. A cross-linking agent comprising the compound according to any one of claims 1 to 4.

6. A composition comprising:
a fluorine-containing elastomer; and
the compound according to any one of claims 1 to 4 or the cross-linking agent according to claim 5.

7. The composition according to claim 6,
wherein the fluorine-containing elastomer contains a cross-linkable group, and
the cross-linkable group includes at least one selected from the group consisting of a hydroxy group (-OH), a cyano group (-CN), a carboxyl group (-COOH), an alkoxycarbonyl group, and an acid halide group.

8. A molded article obtainable by cross-linking the composition according to claim 6 or 7.
